(19) Europäisches Patentamt
European Patent Office
Office européen des brevets

(11) Numéro de publication : **0 502 788 B1**

(12) # FASCICULE DE BREVET EUROPEEN

(45) Date de publication du fascicule du brevet : **11.05.94 Bulletin 94/19**

(21) Numéro de dépôt : **92400578.8**

(22) Date de dépôt : **06.03.92**

(51) Int. Cl.$^5$ : **C07D 251/70,** C07D 401/04, C07D 401/12, C07D 407/14, C07D 409/14, C07D 401/14, A61K 31/53

(54) **Triazines et pyrimidines trisubstituées, leur procédé de préparation et les compositions pharmaceutiques les contenant.**

(30) Priorité : **07.03.91 FR 9102710**

(43) Date de publication de la demande : **09.09.92 Bulletin 92/37**

(45) Mention de la délivrance du brevet : **11.05.94 Bulletin 94/19**

(84) Etats contractants désignés : **AT BE CH DE DK ES FR GB GR IT LI LU NL PT SE**

(56) Documents cités :
**EP-A- 0 090 733**
**EP-A- 0 092 479**
**EP-A- 0 122 855**

(73) Titulaire : **ADIR ET COMPAGNIE**
**1 rue Carle Hébert**
**F-92415 Courbevoie Cédex (FR)**

(72) Inventeur : **Regnier, Gilbert**
**Demeure du Plessis, Bat D, 27 avenue du Plessis**
**F-92290 Chatenay Malabry (FR)**
Inventeur : **Dhainaut, Alain**
**7, rue des Guipières**
**F-78400 Chatou (FR)**
Inventeur : **Atassi, Ghanem**
**4, rue Joséphine**
**F-92400 Saint Cloud (FR)**
Inventeur : **Pierre, Alain**
**52 rue de Montval**
**F-78160 Marly le Roi (FR)**
Inventeur : **Leonce, Stéphane**
**1, rue Lucis**
**F-78000 Versailles (FR)**

(74) Mandataire : **Reverbori, Marcelle**
**Adir et Compagnie, 1, rue Carle Hébert**
**F-92415 Courbevoie Cédex (FR)**

EP 0 502 788 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet les triazines et pyrimidines trisubstituées, leur procédé de préparation et les compositions pharmaceutiques les contenant.

Elle concerne particulièrement les triazines et pyrimidines trisubstituées de formule générale I :

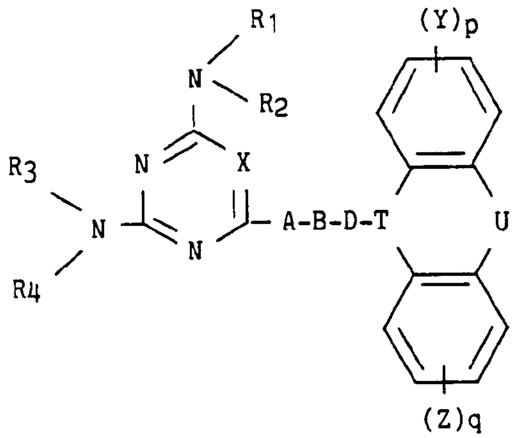

(I)

dans laquelle :

- $R_1$, $R_2$, $R_3$ et $R_4$ : identiques ou différents, représentent chacun : un atome d'hydrogène, un radical cycloalkyle ayant de 3 à 6 atomes de carbone ou un radical alkyle en chaine droite ou ramifiée, ayant de 1 à 6 atomes de carbone, renfermant éventuellement une double ou une triple liaison et éventuellement substitué par un atome d'halogène, un ou plusieurs radicaux hydroxy, ou un reste amino

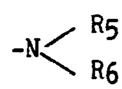,

dans lequel $R_5$ et $R_6$, identiques ou différents, représentent chacun un atome d'hydrogène ou un radical alkyle ayant de 1 à 5 atomes de carbone ou $R_6$ et $R_6$ forment ensemble avec l'atome d'azote auquel ils sont liés un hétérocycle penta- hexa- ou heptagonal renfermant éventuellement un atome d'oxygène ou de soufre ;
- X représente un atome d'azote ou le groupe CH ;
- A représente une liaison simple, un radical hydrocarboné de 1 à 3 atomes de carbone en chaine droite ou ramifiée, ou un groupement -NH-A'- dans lequel A' représente une chaine hydrocarbonée ayant de 2 à 6 atomes de carbone renfermant éventuellement un atome d'oxygène ou de soufre et éventuellement substituée par un radical hydroxy ;
- B représente un radical hétérocyclique de formule :

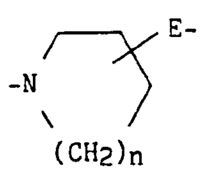

ou

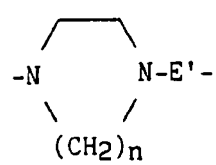

étant défini que :

. n représente un nombre entier de 1 à 3,

. E représente un atome d'oxygène ou de soufre ou un radical -NR- ou $-CH_2NR-$, R étant un atome d'hydrogène ou un radical alkyle ou alkényle ayant chacun jusqu'à 5 atomes de carbone, et

. E' représente une liaison simple ou un radical -NR- tel que défini ci-dessus ;

- D est une liaison simple ou un radical hydrocarboné, en chaine droite ou ramifiée, ayant jusqu'à 6 atomes de carbone ;
- T représente :

. un radical CR' dans lequel R' représente un atome d'hydrogène, un radical alkyle ayant de 1 à 5 atomes de carbone,

. un radical

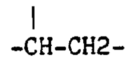

ou

. un atome d'azote ;

- U représente :
  - . une liaison simple,
  - . un radical CHR" dans lequel R" représente un atome d'hydrogène ou un radical alkyle ayant de 1 à 5 atomes de carbone,
  - . un radical di- ou tri-méthylène [$(CH_2)_2$ et $(CH_2)_3$],
  - . un radical -CH=CH,
  - . un atome d'oxygène ou de soufre,
  - . un radical -NR"' dans lequel R"' représente un atome d'hydrogène ou un radical alkyle ayant de 1 à 5 atomes de carbone,
  - . un radical de formule :

    CO, $SO_2$, $-CH_2-O-$, $-CH_2-S-$, $-CH_2-SO_2-$,

    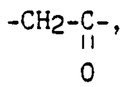, 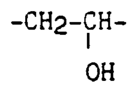 ,

    -CH=N-,

    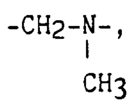, 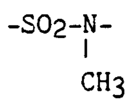 ou 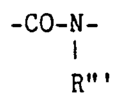

  dans lequel R"' est tel que précédemment défini ; et
- de plus lorsque T et U sont respectivement CR' et CHR" et R' et R" sont différents de H, R' et R" peuvent représenter ensemble un pont polyméthylénique ayant 2 ou 3 atomes de carbone ;
- Y et Z, identiques ou différents représentent chacun un atome d'hydrogène, un atome d'halogène, un radical trifluorométhyle ou un radical alkyle ou alkoxy ayant chacun de 1 à 3 atomes de carbone ; et
- p et q, identiques ou différents prennent chacun les valeurs 1 ou 2 ;

  et, lorsque la formule générale I renferme un ou plusieurs atomes de carbone chiraux, les énantiomères ou diastéréoisomères correspondants.

L'état antérieur de la technique est illustré notamment par le brevet français n° 2.524.467 qui a pour objet les triazines et pyrimidines trisubstituées de formule :

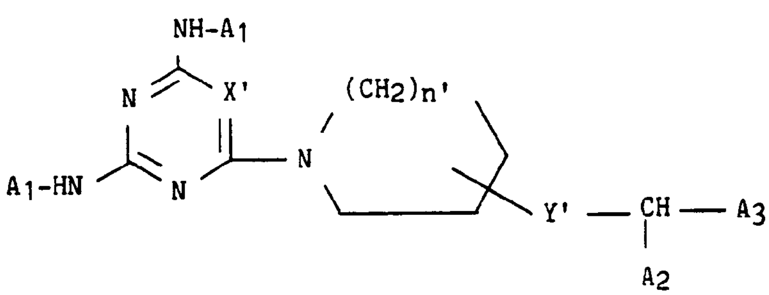

dans laquelle :

- $A_1$ est un radical alkényle de $C_3$ à $C_5$ éventuellement substitué par un ou plusieurs OH ;
- X' représente CH ou N ;

- n' représente zéro, un ou deux ;
- Y' représente O ou N-R'$_1$ [R'$_1$ étant hydrogène, ($C_1$-$C_5$) alkyle ou hydroxyalkyle, ($C_2$-$C_5$) alkényle, ou ($C_3$-$C_7$) - cycloalkyle ou cycloalkényle] ;
- $A_2$ est hydrogène, ($C_1$-$C_5$) alkyle, ($C_5$-$C_7$) cycloalkyle ou phényle éventuellement substitué, et
- $A_3$ est notamment ($C_1$-$C_5$) alkyle, ($C_2$-$C_5$) alkényle, phényle, naphtyle, benzofuranyle, benzothiényle, benzodioxolyle, benzodioxanyle, benzodioxinyle, Δ3 chroményle, thio-chroményle ou chromanyle ;

lesquelles triazines et pyrimidines favorisent la captation d'oxygène et peuvent ainsi être utilisées dans le traitement du déclin cérébral.

Des modifications importantes de structure ont conduit aux dérivés de formule I de la présente invention, lesquels présentent une activité pharmacologique et thérapeutique particulièrement intéressante et totalement différenciée de celle des dérivés proches antérieurement connus, comme le prouve l'étude pharmacologique ci-après décrite.

La présente invention a également pour objet le procédé de préparation des composés de formule générale I caractérisé en ce que l'on condense :

- soit un composé de formule générale II :

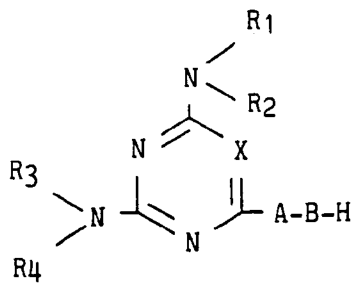

(II)

dans laquelle $R_1$, $R_2$, $R_3$, $R_4$, X, A et B ont les significations précédemment définies, avec un composé de formule générale III :

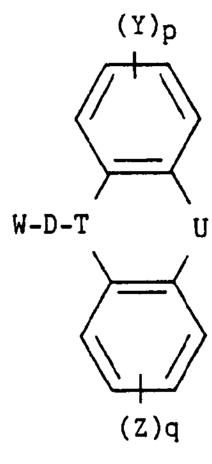

(III)

dans laquelle D, T, U, Y, Z, p et q ont les significations précédemment définies et W représente un atome d'halogène tel que par exemple un atome de chlore ou de brome, ou un reste tosyloxy ;

- soit un composé halogéné de formule générale IV :

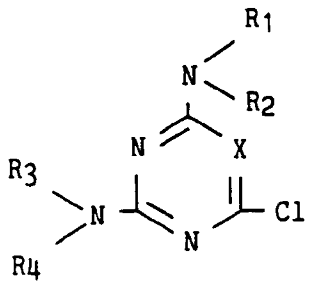

(IV)

dans laquelle $R_1$, $R_2$, $R_3$, $R_4$ et X ont les significations précédemment définies, avec un composé de formule générale V :

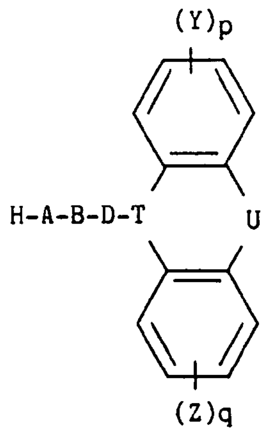

(V)

dans laquelle A, B, D, T, U, Y, Z, p et q ont les significations précédemment définies.

La condensation des composés II et III s'effectue de préférence dans un solvant choisi parmi les alcools contenant 4 ou 5 atomes de carbone, le diméthylformamide, le diméthylacétamide, l'acétonitrile, le tétrahydrofurane, la méthyléthylcétone ou les hydrocarbures aromatiques à haut point d'ébullition.

Il est avantageux d'opérer à une température comprise entre 80 et 120°C, en présence d'un accepteur de l'acide formé au cours de la réaction. Cet accepteur peut être choisi parmi les carbonates alcalins comme le carbonate de potassium, la triéthylamine ou un excès du dérivé II utilisé pour la condensation.

D'autre part, dans le cas où B représente le radical

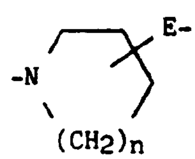

dans lequel n a la signification précédemment définie et E représente uniquement un atome d'oxygène ou de soufre [ce qui entraine pour BH la signification :

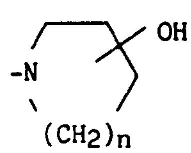 ou 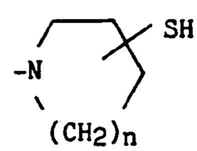 ],

il est judicieux d'employer de l'hydrure de sodium pour soder préalablement le composé II.

La condensation des composés IV et V s'effectue de façon particulièrement adéquate dans un solvant choisi parmi les alcools contenant 4 ou 5 atomes de carbone tel que le butanol ou le pentanol, et les amides aliphatiques comme le diméthylformamide ou le diméthylacétamide. Il est recommandé d'opérer à une température comprise entre 120 et 150°C en présence d'un accepteur de l'hydracide formé au cours de la réaction.

Cet accepteur peut être choisi parmi les carbonates alcalins comme le carbonate de potassium, la triéthylamine ou un excès du composé V utilisé pour la condensation.

De plus, dans le cas où A est une liaison simple et B représente le radical

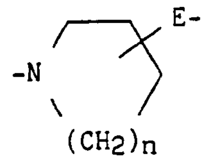

dans lequel n a la signification précédemment définie et E représente uniquement un atome d'oxygène ou de soufre (ce qui entraine pour HAB- la signification :

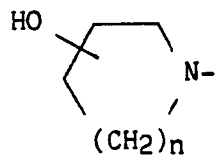 )

il est judicieux d'employer de l'hydrure de sodium pour soder préalablement le composé V.

La présente invention a aussi pour objet le procédé de préparation des composés I dans le cas où B représente le radical

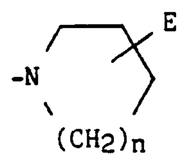

dans lequel n a la signification précédemment définie et E représente un radical -NR c'est à dire plus spécifiquement les composés de formule générale I' :

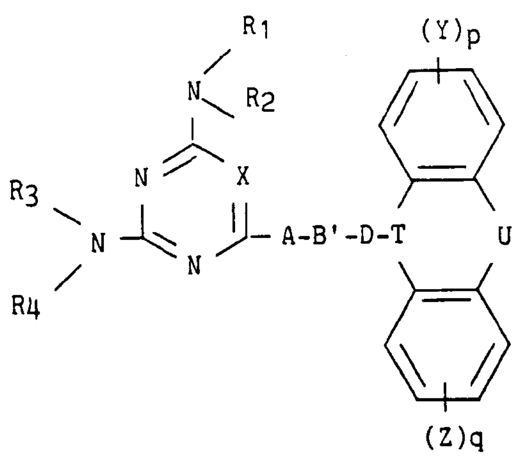

(I')

dans laquelle :

- $R_1$, $R_2$, $R_3$, $R_4$, X, A, D, T, U, Y, Z, p, et q ont les significations précédemment définies, et
- B' représente le radical :

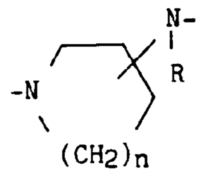

dans lequel n et R ont les significations précédemment définies, caractérisé en ce que l'on condense
. un dérivé de formule générale VI :

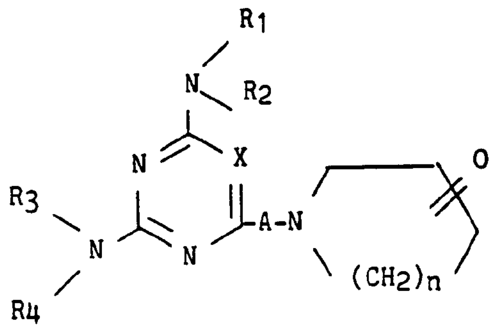

(VI)

dans laquelle $R_1$, $R_2$, $R_3$, $R_4$, X, A et n ont les significations précédemmment défines,
. avec un composé de formule générale VII :

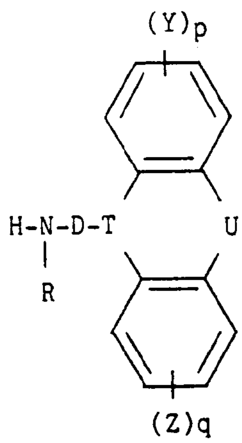

(VII)

dans laquelle R, D, T, U, Y, Z, p et q ont les significations précédemment définies.

Il est particulièrement avantageux d'effectuer la réaction en présence de cyanoborohydrure de sodium, dans un solvant approprié comme les alcools à bas poids moléculaire tel que le méthanol, l'éthanol ou le propanol, ou le tétrahydrofurane, à une température comprise entre 15 et 20°C, à un pH voisin de 6.

Les matières premières utilisées dans les procédés précédemment décrits sont soit des produits connus, soit des produits préparés à partir de substances connues, selon des procédés décrits pour préparer des produits analogues comme indiqué dans les exemples suivants.

Les composés de formule générale I peuvent être transformés en sels d'addition avec les acides, sels qui font, à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthane sulfonique et iséthionique.

D'autre part, dans le cas où la formule générale I renferme un ou plusieurs atomes de carbone chiraux, les dérivés (I) peuvent se présenter sous forme d'énantiomères ou de diastéréoisomères qui font, à ce titre, également partie de l'invention.

Les nouveaux composés (I) peuvent être purifiés par des méthodes physiques telles que cristallisation des bases ou des sels, chromatographie (en particulier chromatoflash sur silice 35-70μ, avec comme système

d'élution : $CH_2Cl_2$ - méthanol ou acétate d'éthyle), ou chimiques telles que formation de sels d'addition avec des acides et décomposition de ces sels par des agents alcalins.

Les composés de formule générale I et leurs sels d'addition physiologiquement tolérables possèdent des propriétés pharmacologiques et thérapeutiques intéressantes, qui permettent de les utiliser pour supprimer la résistance des cellules tumorales aux agents anti-cancéreux et pour supprimer la résistance des parasites aux agents antiparasitaires.

La présente invention a également pour objet les compositions pharmaceutiques contenant comme principe actif, un dérivé de formule générale I ou un de ses sels physiologiquement tolérable, mélangé ou associé à un excipient pharmaceutique approprié.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés, et reste inférieure ou égale à 1 g par prise.

Les exemples suivants illustrent l'invention. Les points de fusion sont déterminés au tube capillaire (cap) ou à la platine chauffante de Kofler (K).

**Exemple 1 :**

2,4-diallylamino-6-{4-(10,11-dihydro-5H-dibenzo (a,d) cycloheptén-5-yl) pipérazin-1-yl}-1,3,5 triazine.

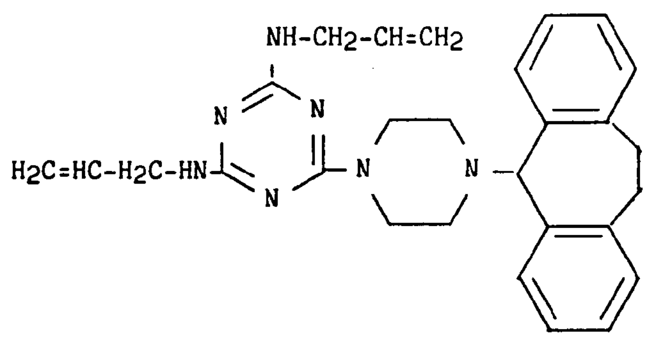

On chauffe à reflux pendant 8 heures une solution de 13,8 g de 2,4-diallylamino-6-(pipérazin-1-yl)-1,3,5 triazine [dont le chlorhydrate fond (K) à 259-263°C] et de 12,6 g de 5-chloro 10,11-dihydro-5H-dibenzo (a,d) cycloheptène [fondant (K) à 110°C] dans 200 ml de toluène, 20 ml de diméthylformamide et 5,56 g de triéthylamine.

Lorsque la réaction est terminée, on traite la solution avec 50 ml d'eau et décante la couche toluénique. Cette opération est renouvelée 2 fois. Les phases toluéniques sont réunies et le toluène est évaporé. Le résidu huileux (26 g) est dissout à l'ébullition dans 150 ml d'éthanol. Le produit cristallise. On isole ainsi 10 g de cristaux de 2,4-diallylamino-6-{4-[(10,11-dihydro-5H-dibenzo (a,d) cycloheptén-5-yl] pipérazin-1-yl}-1,3,5-triazine, P.F. (K) : 192°C.

**Exemple 2 :**

2,4-diallylamino-6-{2-[4-(10,11-dihydro-5H-dibenzo (a,d) cycloheptén-5-yl) pipérazin-1-yl] éthylamino}-1,3,5-triazine.

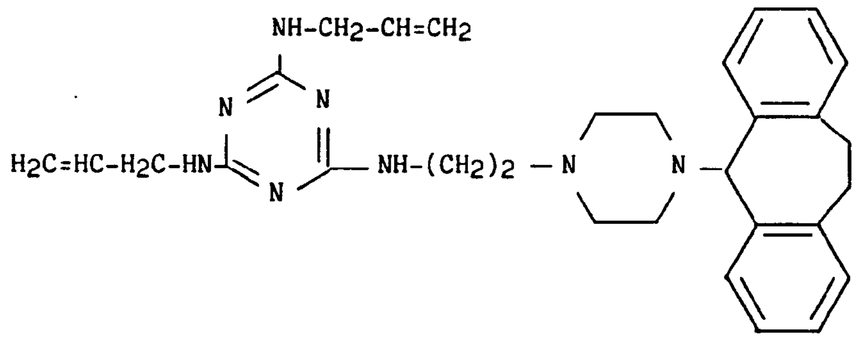

On chauffe à reflux pendant 12 heures une solution de 3,2 g de 2,4-diallylamino-6 chloro-1,3,5 triazine, fondant (K) à 204°C, et de 4,5 g de 1-aminoéthyl-4-(10,11-dihydro-5H-dibenzo (a,d) cycloheptén-5-yl) pipérazine, fondant (cap) à 70-71°C, dans 100 ml de butanol, en présence de 1,9 g de carbonate de potassium et 0,1 g d'iodure de potassium.
Lorsque la réaction est terminée, on filtre le sel, évapore le solvant et reprend le résidu avec de l'éther. On lave à l'eau et sèche sur $MgSO_4$. Après évaporation de l'éther, le résidu huileux est chromatographié sur 120 g de silice, en éluant avec le système $CH_2Cl_2$-$CH_3OH$ (92-8).
Après évaporation des éluats, on transforme la base récupérée en difumarate au sein de l'éthanol. On obtient finalement 7,7 g de cristaux de difumarate de 2,4-diallylamino-6-{2-[4-(10,11-dihydro-5H-dibenzo (a,d) cycloheptén-5-yl) pipérazin-1-yl] éthylamino}-1,3,5-triazine fondant (cap) à 123-128°C.
L'aminoéthylpipérazine de départ a été préparée par réduction du dérivé cyanométhylé correspondant, [P.F. (cap) : 112-113°C] par $H_2$/Ni , au sein de l'éthanol, en présence de $NH_3$.

**Exemple 3 :**

2,4-diallylamino-6-{4-[(10,11-dihydro-5H-dibenzo (a,d) cycloheptén-5-yl) méthylamino] pipéridin-1-yl}-1,3,5-triazine.

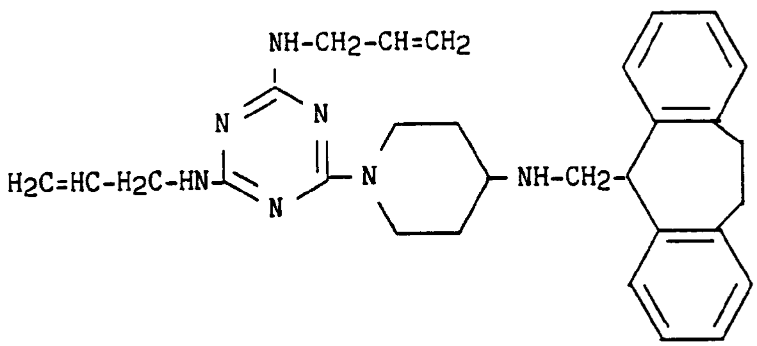

A une solution de 20,5 g de chlorhydrate de 5-aminométhyl 10,11-dihydro-5H-dibenzo (a,d) cycloheptène, fondant (cap) à 270-280°C, on ajoute à 10°C, une solution de méthylate de sodium préparée extemporanément à partir de 1,55 g de sodium. Lorsque la solution est homogène, on ajoute 16,2 g de chlorhydrate de 1-(4,6-diallylamino-1,3,5-triazin-2-yl) pipérid-4-one, fondant (cap) à 219-222°C puis 5 g de cyanoborohydrure de sodium ; la température étant toujours maintenue à 10°C. Le pH de la solution est amené à 6 par addition de méthanol chlorhydrique et la solution est agitée pendant 24 heures à température ambiante, en présence de tamis moléculaire 3Å.
Lorsque la réaction est terminée, le sel insoluble est filtré et le filtrat concentré sous pression réduite.
Le résidu est alors repris avec 150 ml de $CH_2Cl_2$ et lavé avec 2 fois 100 ml d'une solution à 10 % de $NaHCO_3$ puis lavé à l'eau et enfin séché sur $Na_2SO_4$.
Après évaporation du solvant, l'huile obtenue est chromatographiée sur 1 kg de silice (35-70μ) en éluant avec le système $CH_2Cl_2$-$CH_3OH$ (95-5).
Après évaporation des éluats, le produit est recristallisé dans l'éther. On obtient 17,5 g de 2,4-diallylamino-6-{4-[(10,11-dihydro-5H-dibenzo (a,d) cycloheptén-5-yl) méthylamino] pipéridin-1-yl}-1,3,5 triazine, sous forme de cristaux blancs fondant (cap) à 131-134°C.
La 1-(4,6-diallylamino-1,3,5-triazin-2-yl) pipérid-4-one de départ a été préparée par hydrolyse acide du diéthylacétal correspondant, lui-même préparé par condensation de la 4,6-diallylamino-2-chloro-1,3,5-triazine, fondant (cap) à 206°C, avec la 4,4-diéthoxy pipéridine, dans le butanol à reflux.

La 2,4-diallylamino-6-{4-[(10,11-dihydro-5H-dibenzo(a,d) cycloheptén-5-yl) méthylamino] pipéridin-1-yl}-1,3,5-triazine a également été préparée selon la méthode décrite dans l'exemple 1.

**Exemples 4 à 32:**

En appliquant une ou plusieurs des méthodes de préparation décrites dans les exemples 1 à 3, ont été préparés les composés suivants :

**4.** 2,4-diallylamino-6-[4-(10,11-dihydro-5H-dibenzo (a,d) cycloheptén-5-yl amino) pipéridin -1-yl]-1,3,5-triazine, P.F. (cap) du fumarate correspondant : 187-190°C, selon l'exemple 1 et l'exemple 3.

**5.** 2-allylamino-4-propylamino-6-{4-[(10,11-dihydro-5H-dibenzo (a,d) cycloheptén-5-yl) méthylamino] pipéridin-1-yl}-1,3,5-triazine, P.F. (cap) : 118-121°C, selon l'exemple 1 et l'exemple 3.

**6.** 2,4-diallylamino-6-{3-[4-(10,11-dihydro-5H-dibenzo (a,d) cycloheptén-5-yl) pipérazin-1-yl]-2-hydroxypropylamino}-1,3,5-triazine, produit amorphe, selon l'exemple 2.

**7.** 2,4-diallylamino-6-{[1-(10,11-dihydro-5H-dibenzo (a,d) cycloheptén-5-yl) pipéridin-4-yl] méthylamino}-1,3,5-triazine, produit amorphe, selon l'exemple 2.

**8.** 2,4-diallylamino-6-{1-[(10,11-dihydro-5H-dibenzo (a,d) cycloheptén-5-yl) méthyl] pipéridin-4-yl] méthylamino}-1,3,5-triazine, produit amorphe, selon l'exemple 2.

**9.** 2,4-diallylamino-6-[4-(xanthén-9-yl méthylamino) pipéridin-1-yl]-1,3,5 triazine, P.F. (cap) : 70-77°C, selon l'exemple 3.

**10.** 2,4-diallylamino-6-{4-[(9,10-dihydro-9-10-éthanoanthracén-9-yl) méthylamino] pipéridin-1-yl}-1,3,5-triazine, P.F. (cap) : 148-150°C, selon l'exemple 3.

**11.** 2,4-diallylamino-6-{4-[(5H-dibenzo (a,d) cycloheptén-5-yl) méthylamino] pipéridin-1-yl}-1,3,5 triazine, P.F. (cap) du difumarate correspondant : 203-205°C, selon l'exemple 3.

**12.** 2,4-diallylamino-6-{2-[4-(10,11-dihydro-5H-dibenzo (a,d) cycloheptén-5-yl amino) pipéridin-1-yl] éthylamino}-1,3,5-triazine, P.F.(cap) du difumarate correspondant : 128-131°C, selon l'exemple 3.

**13.** 2,4-diallylamino-6-{4-[(fluorén-9-yl) méthylamino] pipéridin-1-yl}-1,3,5-triazine, P.F. (cap) du difumarate correspondant : 139-144°C, selon l'exemple 3.

**14.** 2,4-diméthylamino-6-{4-[(10,11-dihydro-5H-dibenzo (a,d) cycloheptén-5-yl) méthylamino] pipéridin-1-yl}-1,3,5-triazine, P.F. (cap) du fumarate correspondant : 234°C, selon l'exemple 3.

**15.** 2,4-diallylamino-6-{2-[4-(10,11-dihydro-5H-dibenzo (a,d) cycloheptén-5-yl méthylamino) pipéridin-1-yl] éthylamino}- 1,3,5-triazine, P.F. (cap) du difumarate correspondant : 212°C, selon l'exemple 3.

**16.** 2,4-diallylamino-6-{3-[4-(10,11-dihydro-5H-dibenzo (a,d) cycloheptén-5-yl amino) pipéridin-1-yl]-2-hydroxypropylamino}-1,3,5-triazine, P.F. (cap) du difumarate correspondant : 148-151°C, selon l'exemple 3.

**17.** 2,4-diallylamino-6-{3-[4-(10,11-dihydro-5H-dibenzo (a,d) cycloheptén-5-yl méthylamino) pipéridin-1-yl]-2-hydroxypropylamino}-1,3,5-triazine, P.F. (cap) du difumarate correspondant : 170-173°C, selon l'exemple 3.

**18.** 2-allylamino-4-amino-6-{4-[(10,11 dihydro-5H-dibenzo (a,d) cycloheptén-5-yl) méthylamino] pipéridin-1-yl}-1,3,5-triazine, P.F. (cap) du dimaléate correspondant : 153-155°C, selon l'exemple 3.

**19.** 2,4-diallylamino-6-{4-[((R,S)-6,11 dihydro-dibenzo (b,e) oxépin-11-yl) méthylamino] pipéridin-1-yl}-1,3,5-triazine, P.F. (cap) : 95-97°C, selon l'exemple 3.

**20.** 2,4-diallylamino-6-{4-[(5,6,7,12-tétrahydro dibenzo (a,d) cycloctén-12-yl) méthylamino] pipéridin-1-yl}-1,3,5-triazine, P.F. (cap) : 122°C, selon l'exemple 3.

**21.** 2,4-diallylamino-6-{4-[(8-chloro-10,10-dioxo-11 méthyl (R,S) dibenzo (c,f) thiazépin-5-yl) méthylamino] pipéridin-1-yl}-1,3,5-triazine, P.F. (cap) : 153-155°C, selon l'exemple 3.

**22.** 2-allylamino-4-éthylamino-6-{4-[(10,11-dihydro-5H-dibenzo (a,d) cycloheptén-5-yl) méthylamino] pipéridin-1-yl}-1,3,5-triazine, P.F. (cap) : 120-122°C, selon l'exemple 3.

**23.** 2,4-diallylamino-6-{4-[(10, 11-dihydro-5H-dibenzo (a,d) cycloheptén-5-yl) aminométhyl] pipéridin-1-yl}-1,3,5-triazine, P.F. (cap) : 109-110°C, selon l'exemple 3.

**24.** 2,4-diallylamino-6-{4-[(10,11-dihydro-5H-dibenzo (a,d) cycloheptén-5-yl) méthylamino] pipérazin-1-yl}-1,3,5-triazine, P.F. (cap) du fumarate correspondant : 138-142°C.

**25.** 2,4-diallylamino-6-{4-[(10,11-dihydro-5H-dibenzo (a,d) cycloheptén-5-yl) méthyl] pipérazin-1-yl}-1,3,5-triazine, P.F. (cap) : 104-105°C, selon l'exemple 1.

**26.** 2,4-diallylamino-6-{4-[((R,S)-6,11-dihydro-dibenzo (b,e) thiépin-11-yl) méthylamino] pipéridin-1-yl}-1,3,5-triazine, PF (cap) : 131-132°C, selon l'exemple 3.

**27.** 2,4-diallylamino-6-{4-[((R,S)-6,11-dihydro-dibenzo (b,e)-6-oxoazépin-11-yl) méthylamino] pipéridin-1-yl}-1,3,5-triazine, PF (cap) : 185-187°C, selon l'exemple 3.

**28.** 2-allylamino-4-méthylamino-6-{4-[((R,S)-6,11-dihydro-dibenzo (b,e)-oxépin-11-yl) méthylamino] pipéridin-1-yl}-1,3,5-triazine, PF (cap) : 113-116°C, selon l'exemple 3.

**29.** 2-allylamino-4-éthylamino-6-{4-[((R,S)-6,11-dihydro-dibenzo (b,e)-oxépin-11-yl) méthylamino] pipéridin-1-yl}-1,3,5-triazine, PF (cap) : 106-107°C, selon l'exemple 3.

**30.** 2,4-diallylamino-6-{4-[N-(10,11-dihydro-5H-dibenzo (a,d)-cycloheptén-5-yl) méthyl)-N-méthylamino] pipéridin-1-yl}-1,3,5-triazine, PF (cap) : 105-107°C, selon l'exemple 3.

**31.** 2,4-diallylamino-6-{4-[((R,S)-10,11-dihydro-5H-2-méthoxydibenzo (a,d)-cycloheptén-5-yl) méthylamino] pipéridin-1-yl}-1,3,5-triazine, PF (cap) du difumarate correspondant : 184-187°C, selon l'exemple 3.

**32.** 2,4-diallylamino-6-{4-[((R,S)-6,11-dihydro-5,5-dioxo-dibenzo (b,e)-thiépin-11-yl) méthylamino] pipéridin-1-yl}-1,3,5-triazine, PF (cap) : 116-119°C, selon l'exemple 3.

Les matières premières, autres que celles mentionnées dans les exemples 1 à 3, sont répertoriées dans les tableaux ci-après.

TABLEAU A

Composés de formule II :

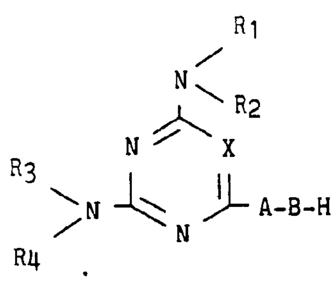

| $R_1$ | $R_2$ | $R_3$ | $R_4$ | X | A | B | P.F °C |
|---|---|---|---|---|---|---|---|
| H | allyl | H | propyl | N | — | 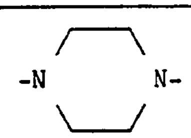 | 100 (K) |
| H | allyl | H | allyl | N | — | 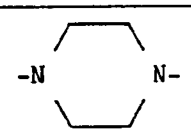 | 259-263 (K) |
| H | allyl | H | allyl | N | — | 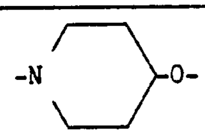 | 118-119 (cap) |
| H | allyl | H | allyl | N | — | 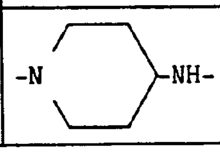 | 218-220 (cap) |

TABLEAU B

Composés de formule III :

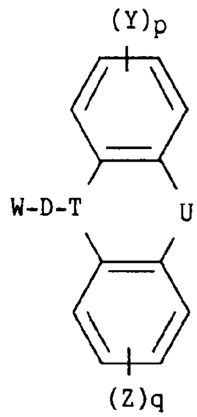

| W | D | T | U | $(Y)_p$ | $(Z)_q$ | P.F °C |
|---|---|---|---|---|---|---|
| Cl | — | CH | $(CH_2)_2$ | H | H | 110 (K) |
| Cl | $-CH_2-$ | CH | — | H | H | 108 (K) |

TABLEAU C

Composés de formule IV :

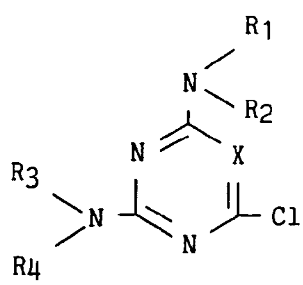

| $R_1$ | $R_2$ | $R_3$ | $R_4$ | X | P.F °C |
|---|---|---|---|---|---|
| H | allyl | H | H | N | 161-162 (cap) |
| H | allyl | H | allyl | N | 206-208 (cap) |
| H | allyl | H | propyl | N | 210 (K) |
| H | allyl | H | allyl | CH | 204 (K) |

TABLEAU D

Composés de formule V :

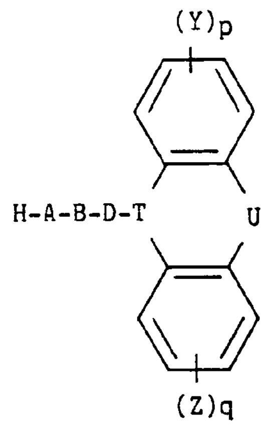

| A | B | D | T | U | (Y)p | (Z)q | P.F. °C |
|---|---|---|---|---|---|---|---|
| — | 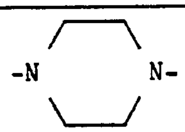 | — | CH | $(CH_2)_2$ | H | H | 99-100 (cap) |
| $HN-(CH_2)_2-$ | 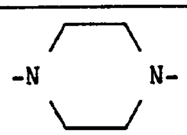 | — | CH | $(CH_2)_2$ | H | H | 70-71 (cap) |
| $HN-CH_2-\underset{\mid\atop OH}{CH}-CH_2$ | 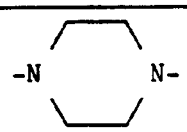 | — | CH | $(CH_2)_2$ | H | H | 100 (cap) |

TABLEAU E

Composés de formule VI :

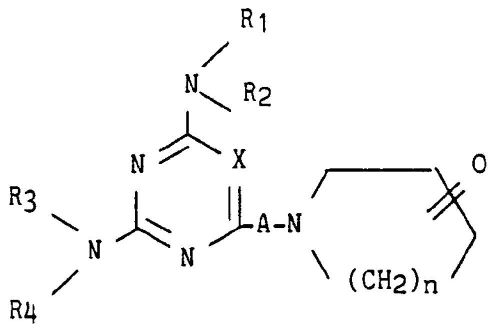

| $R_1$ | $R_2$ | $R_3$ | $R_4$ | X | A | 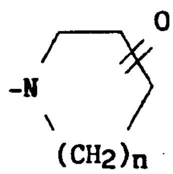 | P.F °C |
|---|---|---|---|---|---|---|---|
| H | H | H | H | N | — | 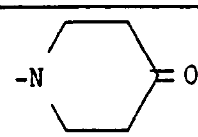 | HCl, 214 (K) |
| $CH_3$ | $CH_3$ | $CH_3$ | $CH_3$ | N | — | 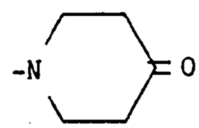 | 2HCl, 144 (K) |
| H | allyl | H | propyl | N | — | 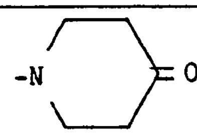 | HCl, 220 (K) |
| H | allyl | H | allyl | N | — | 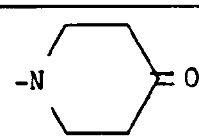 | HCl, 219-222 (cap) |
| H | allyl | H | allyl | N | — | 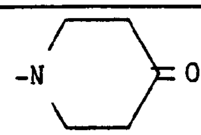 | 232 (K) |

TABLEAU E (suite)

| R1 | R2 | R3 | R4 | X | A | 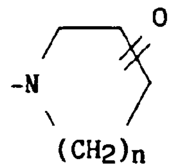 | P.F °C |
|---|---|---|---|---|---|---|---|
| H | allyl | H | allyl | N | — | 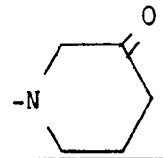 | HCl, 192 (K) |
| H | allyl | H | allyl | N | $-NH-(CH_2)_2-$ | 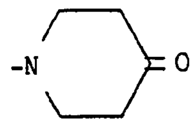 | amorphe |
| H | allyl | H | allyl | N | $-NH-CH-CH_2-$<br>OH | 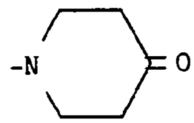 | amorphe |
| H | allyl | H | allyl | CH | — | 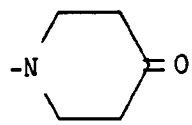 | fumarate, 240 (cap) |

TABLEAU F

Composés de formule VII :

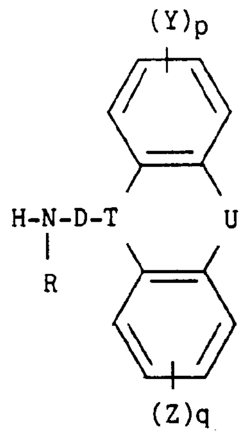

| R | D | T | U | $(Y)_p$ | $(Z)_q$ | P.F °C |
|---|---|---|---|---|---|---|
| H | $CH_2$ | CH | $(CH_2)_2$ | H | H | HCl, 270-280 (cap) |
| H | $CH_2$ | CH | CH=CH | H | H | HCl, 276 (cap) |
| H | $CH_2$ | CH | O | H | H | HCl, 230-235 (cap) |
| H | $CH_2$ | CH | — | H | H | HCl, 272 (cap) |
| H | $CH_2$ | $-C-(CH_2)_2-CH$ | | H | H | 51-55 (cap) |

Exemple 33 :

ETUDE PHARMACOLOGIQUE :

La résistance aux agents anticancéreux est un obstacle majeur à l'efficacité des drogues antitumorales. Parmi les différents types de résistance, la "Multidrug résistance" (MDR) est particulièrement intéressante, puisqu'elle est induite par des composés d'origine naturelle, actifs contre les tumeurs solides (Anthracyclines, Vinca alcaloides, Epipodophyllotoxines par exemple) et qu'elle est très fréquente dans certains cancers (colon par ex.). Les cellules tumorales, lorsqu'elles sont exposées in vitro ou in vivo à l'une de ces drogues, deviennent résistantes, à divers degrés, à l'ensemble de ces composés. Le phénomène de résistance est dû à l'action d'une protéine membranaire inductible, la gP 170, dont le rôle est d'augmenter l'efflux du cytotoxique, donc de diminuer sa concentration intracellulaire, d'où la perte de sensibilité de ces cellules à la drogue.
Des médicaments, utilisés dans d'autres pathologies, sont connus pour reverser, partiellement ou totalement,

cette résistance (Tsuruo T., Mechanisms of multidrug résistance and implications for therapy. Int. J. Cancer Res., 79, 285-296,1988 ; Rothenberg, M and Ling V., Multidrug résistance : molecular biology and clinical relevance, J.N.C.I.,81, 907-910, 1989 ; Gottesman M.M. and Pastan, I., résistance to multiple chemotherapeutic agents in human cancer cells, Trends Pharmacol. Sci, 9, 54-58, 1989 ; Endicott J.A. and Ling V., The biochemistry of P-glycoprotein-mediated multidrug résistance, Annu. Rev. Biochem., 58, 137-171, 1989.).
L'agent modulateur, lorsqu'il est ajouté en même temps que le cytotoxique, diminue, on supprime totalement la résistance de type MDR. Certains médicaments, comme le vérapamil, l'amiodarone ou la cyclosporine ont été utilisés en clinique pour lever cette résistance, mais leurs propriétés pharmacologiques intrinsèques et leurs toxicités limitent considérablement leur utilisation. D'où l'intérêt de la recherche de composés réversant le phénotype MDR tout en étant dépourvus d'autres propriétés pharmacologiques et de toxicité.
De plus, le mécanisme de la résistance à la chloroquine, développée par Plasmodium falciparum, est similaire. Le vérapamil restaure la sensibilité d'une lignée résistante (Krogstad D.J., Gluzman I.Y., Kule D.E., Oduola A.M.J., Martin S.K., Milhous W.K., Schlessinger P.H.., Efflux of Chloroquine from Plasmodium falciparum : mechanism of chloroquine résistance. Science, 238, 1283-1285, 1987 ; Martin S.K., Oduola A.M.J., Milhous W.K., Reversal of Chloroquine résistance in Plasmodium falciparum by Verapamil, Science, 235, 899-901, 1987.), ce qui démontre l'intérêt potentiel de composés réversant le phénotype MDR des cellules tumorales pour une utilisation en parasitologie.
L'étude pharmacologique des composés de la présente invention a consisté tout d'abord en une évaluation in vitro effectuée sur des cellules résistantes.
Le paramètre mesuré est la cytotoxicité de la drogue antitumorale, quantifiée en absence et en présence du composé reversant .
On a également mesuré l'effet des produits sur la concentration intracellulaire du cytotoxique.
En effet, les composés connus pour réverser la MDR agissent en augmentant la concentration intracellulaire en cytotoxique. Cet effet est la conséquence de l'inhibition de l'action de la gP 170 responsable de l'efflux de la drogue.
Cette étude a été complétée par une étude in vivo, en utilisant une leucémie murine résistante à la vincristine (P/388/VCR) et en associant les produits à la vincristine.

Matériel et méthodes :

1) Activité in vitro

**Cytotoxicité**

Deux lignées cellulaires résistantes ont été utilisées :

- 1 Carcinome épidermoïde humain, KB-A1 dont la résistance a été induite par l'adriamycine (ADR). Son facteur de résistance est de 200, par rapport à la lignée sensible (résistance moyenne).
- 1 lignée de poumon de hamster chinois, DC-3F/AD, dont la résistance a été induite par l'actinomycine D. Son facteur de résistance est supérieur à 10 000, c'est donc une lignée extrêmement résistante. Ces deux lignées sont résistantes également aux Vinca alcaloïdes (Vincristine et Vinblastine).

Les cellules sont cultivées dans-du milieu de culture (RPMI 1640) complet contenant 10 % de sérum de veau foetal, 2 mM de glutamine ; 50 unités/ml de pénicilline, 50 ug/ml de streptomycine, 10 mM d'Hepes.
Les cellules sont réparties dans des microplaques et exposées aux composés cytotoxiques (Actinomycine D pour la lignée DC-3F/AD et Adriamycine pour la lignée KB-A1) à 9 concentrations (dilutions sériées de 2 en 2). Les produits testés pour leur capacité à réverser la MDR sont ajoutés en même temps que le cytotoxique. Les cellules sont ensuite incubées pendant 4 jours. Le nombre de cellules viables est ensuite quantifié par un essai colorimétrique, le Microculture Tétrazolium Assay (Carmichael J., DeGraff W.G., Gazdar A.F., Minna J.D. and Mitchell J.R. Evaluation of a tetrazolium-based semiautomated colorimetric assay : assessment of chemosensitivity testing, Cancer Res, 47, 936-942, 1987.). Les résultats sont exprimés en IC50, concentration en cytotoxique qui inhibe à 50 % la prolifération des cellules témoins. Les résultats sont exprimés en facteur de réversion (FR), avec

$$FR = \frac{\text{IC50 cytotoxique seul}}{\text{IC50 cytotoxique en présence du composé réversant}}$$

**Cytométrie en flux**

Certains composés anticancéreux comme l'adriamycine (ADR) ont la propriété d'être fluorescents après excitation par une source lumineuse de longueur l'onde connue.

Par la mesure de cette fluorescence, il est ainsi possible de mesurer de façon relative la concentration intracellulaire en ADR. La cytométrie en flux (CMF) est un outil de choix pour effectuer ce type de mesure et ainsi déterminer rapidement si certains composés actifs agissent en augmentant la concentration intracellulaire en ADR.
Les cellules $500.10^3$ par ml ont été exposées simultanément à l'ADR à une concentration fixe (50µM) et aux composés testés à différentes concentrations. Après 5 heures d'incubation, l'accumulation de l'ADR intra-cellulaire a été évalué par CMF. Les analyses ont été réalisées sur un cytomètre en flux ATC3000 (BRUKER-FRANCE) équipé d'un laser argon 2025 (SPECTRA-PHISICS-FRANCE) optimisé à 488 nm pour une puissance de 600 mW.
L'analyse de chacun des échantillons a été effectuée sur un total de 10 000 cellules à une vitesse de 1 000 cellules par seconde.
Les résultats ont été collectés sous forme d'histogrammes linéaires de la fluorescence de l'ADR intra-cellulaire.
Expression des résultats : Pour chacun des histogrammes, le canal moyen (MEAN) de fluorescence a été déterminé par le système informatique de l'appareil. Pour toutes les expériences :

- un contrôle négatif (cellules sans ADR) a fixé le seuil d'autofluorescence.
- un contrôle positif (cellules avec ADR) a déterminé la valeur MEAN=MN1.
- les tubes "tests" (cellules avec ADR et avec produit) ont déterminé pour chacun des produits et à chacune des concentrations les valeurs MEAN = MN2.

Les résultats sont exprimés sous forme de variations de la moyenne de fluorescence obtenue pour chacun des tubes "tests" (MN2) par rapport à la moyenne de fluorescence obtenue avec le contrôle positif (MN1) : VAR-MEAN = MN2-MN1. Le paramètre exprimé est donc l'augmentation de la fluorescence de l'ADR en présence des composés testés.

2) Activité in vivo

**Activité antitumorale**

La lignée sensible parentale P 388 (leucémie murine) et la sous-lignée résistante à la vincristine, P 388/VCR, ont été fournies par le NCI (Frederick, USA). Les cellules tumorales ($10^6$ cellules) ont été inoculées au jour zéro dans la cavité intrapéritonéale à des souris B6D2F1 femelles (Iffa Credo, France) pesant de 18 à 20 g (groupes de 8 à 10 animaux).
Les animaux ont reçu tous les jours pendant 4 jours, à partir du jour 1 :

- une administration de produit de la présente invention à tester à raison de 50 ou 100 mg/kg par voie i.p., puis
- 30 à 60 minutes après, une administration de vincristine (pris comme agent anti-tumoral de référence) à raison de 0,25 mg/kg par voie i.p.

L'activité tumorale est exprimée en :

$$\frac{T}{C}\% = \frac{\text{Temps de survie médian des animaux traités}}{\text{Temps de survie médian des animaux contrôles}} \times 100$$

Les valeurs sont des moyennes obtenues dans des expériences indépendantes (± sem quand n est supérieur ou égal à 3).

Résultats :

1) Activité in vitro

**Cytotoxicité**

Le tableau 1 donne les valeurs des facteurs de réversions obtenus avec les différents composés avec la lignée DC-3F/AD et le tableau 2 avec la lignée KB-A1.

**Cytométrie en flux**

Le tableau 3 donne l'augmentation de la fluorescence de l'ADR (VAR-MEAN) obtenue avec les différents composés sur la lignée DC-3F/AD et le tableau 4 avec la lignée KB-A1.

2) Activité in vivo

Le tableau 5 montre l'augmentation de l'activité antitumorale in vivo de la vincristine obtenue avec différents composés représentatifs de la présente invention.
Tous les produits des exemples de l'invention testés augmentent considérablement l'activité antitumorale de la vincristine dans des cellules résistantes, et sont, pour la plupart, plus actifs que le vérapamil.

TABLEAU 1

Facteurs de réversion (cytotoxicité) avec la lignée DC-3F/AD

| PRODUITS | CONCENTRATIONS | | | |
|---|---|---|---|---|
| | 0,5 µM | 1 µM | 2,5 µM | 5 µM |
| PRODUIT DE REFERENCE | | | | |
| VERAPAMIL | 0,9 | 0,9 | 1,2 | 2,5 |
| PRODUIT DES EXEMPLES | | | | |
| 1 | - | 27 | 142 | 309 |
| 2 | 0,8 | 1 | 22 | - |
| 3 | 43 | 236 | 1474 | 3187 |
| 4 | 21 | 66 | 346 | 755 |
| 5 | 3,8 | 81 | 906 | 2753 |
| 6 | 0,9 | 1,1 | 44 | - |
| 7 | 1,2 | 1,1 | 1,6 | 9,7 |
| 8 | 0,8 | 1,6 | 50 | 353 |
| 9 | 4,8 | 39 | 251 | 844 |
| 10 | 1 | 1,9 | 23 | 96 |
| 11 | 2 | 26 | 364 | 1408 |
| 12 | 0,9 | 0,9 | 2,4 | 64 |
| 13 | 2,1 | 7,1 | 36 | 170 |
| 14 | 1,3 | 7,8 | 47 | 187 |
| 15 | 0,8 | 1 | 1,4 | 120 |
| 16 | 1 | 0,9 | 1 | 1,8 |
| 17 | 0,8 | 0,8 | 0,9 | 1,1 |
| 18 | 1,2 | 3 | 64 | 421 |
| 19 | 3,8 | 63 | 549 | 2525 |
| 20 | 3,4 | 52 | 447 | 2951 |
| 21 | 1,2 | 18 | 883 | 2804 |
| 22 | 32 | 177 | 1417 | 2499 |
| 23 | - | 1,4 | 49 | 173 |
| 24 | 2,5 | 14 | 79 | 329 |
| 25 | - | 13 | 127 | 464 |
| 26 | - | 6,8 | 168,9 | 866,6 |
| 27 | - | 1,1 | 1,3 | 2,1 |
| 28 | - | 18,1 | 113,8 | 600,4 |
| 29 | 1,7 | 33,8 | 266,9 | 1327,5 |
| 30 | - | 2,3 | 59,8 | 834,0 |
| 31 | 3,1 | 85,9 | 761,3 | 1913,4 |
| 32 | - | 1,3 | 1,9 | 27,4 |

TABLEAU 2

Facteurs de réversion (cytotoxicité) avec la lignée KB-A1

| PRODUITS | CONCENTRATIONS | | | |
|---|---|---|---|---|
| | 0,5 µM | 1 µM | 2,5 µM | 5 µM |
| PRODUIT DE REFERENCE | | | | |
| VERAPAMIL | 1,5 | 5,8 | 23 | 27 |
| PRODUIT DES EXEMPLES | | | | |
| 1 | 1,7 | 4 | 16 | 36 |
| 2 | 0,8 | 1,4 | 32 | - |
| 3 | 91 | 131 | 217 | 252 |
| 4 | 42 | 54 | 99 | 142 |
| 5 | 119 | 153 | 258 | 221 |
| 6 | 1,1 | 1,2 | 26 | 18 |
| 7 | 1,8 | 1,7 | 2,3 | 10 |
| 8 | 1,8 | 7,8 | 29 | 12 |
| 9 | 72 | 96 | 163 | 140 |
| 10 | 2,3 | 6,4 | 20 | - |
| 11 | 156 | 228 | 212 | 641 |
| 12 | 1,2 | 3 | 63 | |
| 13 | 16 | 17 | 22 | 57 |
| 14 | 13 | 17 | 20 | 6,9 |
| 15 | 0,8 | 1 | 13 | - |
| 16 | 0,8 | 0,9 | 11 | - |
| 17 | 0,8 | 0,8 | 1,2 | 6,8 |
| 18 | 4,9 | 13 | 49 | 89 |
| 19 | 132 | 153 | 228 | 270 |
| 20 | 35 | 66 | 92 | 177 |
| 21 | 22 | 36 | 93 | 147 |
| 22 | 86 | 116 | 145 | 201 |
| 23 | 18 | 48 | 92 | 126 |
| 24 | 13 | 24 | 41 | 93 |
| 25 | 7,1 | 15 | 28 | 63 |
| 26 | 60,2 | 122,1 | 246,7 | 393,5 |
| 27 | 15,3 | 84,8 | 189,4 | 314,7 |
| 28 | 88,0 | 93,1 | 158,2 | 183,1 |
| 29 | 132,7 | 147,0 | 277,0 | 229,1 |
| 30 | 12,3 | 27,7 | 71,2 | 117,6 |
| 31 | 121,8 | 175,8 | 258,9 | 263,9 |
| 32 | 17,8 | 44,3 | 109,2 | 252,9 |

TABLEAU 3

Mesure de l'accumulation intracellulaire en adriamycine par les cellules de la lignée DC-3F/AD

| PRODUITS | VAR-MEAN AUX CONCENTRATIONS DE | | | |
|---|---|---|---|---|
| | 1.0 µM | 2.5 µM | 5.0 µM | 10 µM |
| PRODUIT DE REFERENCE | | | | |
| VERAPAMIL | 2,1 | 4,0 | 5,6 | 8,9 |
| PRODUIT DES EXEMPLES | | | | |
| 1 | 8,4 | 4,9 | 16,0 | 19,3 |
| 2 | 10,1 | 14,7 | 10,2 | 10,5 |
| 3 | 20,2 | 25,8 | 28,3 | 29,6 |
| 4 | 16,2 | 21,8 | 25,5 | 28,1 |
| 5 | 17,9 | 23,5 | 27,3 | 24,9 |
| 6 | 11,0 | 14,4 | 24,3 | 27,6 |
| 7 | 3,4 | 3,9 | 10,1 | 17,4 |
| 8 | 1,3 | 11,4 | 19,3 | 21,0 |
| 9 | 13,4 | 18,1 | 19,4 | 25,7 |
| 10 | 12,3 | 13,5 | 20,2 | 30,7 |
| 11 | 12,7 | 20,2 | 24,5 | 25,8 |
| 12 | 5,4 | 10,3 | 10,4 | 18,3 |
| 13 | 8,1 | 12,0 | 19,6 | 23,2 |
| 14 | 12,3 | 14,5 | 29,4 | 30,6 |
| 15 | 10,1 | 12,4 | 13,7 | 12,4 |
| 16 | 9,9 | 11,8 | 10,4 | 12,9 |
| 17 | 9,4 | 11,0 | 10,5 | 12,6 |
| 18 | 5,5 | 7,4 | 6,7 | 11,0 |
| 19 | 13,1 | 22,6 | 25,5 | 25,3 |
| 20 | 8,2 | 19,7 | 22,3 | 21,6 |
| 21 | 8,1 | 21,1 | 22,8 | 24,1 |
| 22 | 15,1 | 21,9 | 23,1 | 24,9 |
| 23 | 10,0 | 14,6 | 14,0 | 20,5 |
| 24 | 12,8 | 16,9 | 21,1 | 28,2 |
| 25 | 9,9 | 14,7 | 17,6 | 24,5 |
| 26 | 4,3 | 17,8 | 33,5 | 32,6 |
| 27 | 0,0 | 0,0 | 2,2 | - |
| 28 | 4,3 | 12,8 | 16,8 | 21,5 |
| 29 | 6,4 | 13,3 | 18,0 | 21,8 |
| 30 | 5,9 | 12,3 | 17,8 | 19,0 |
| 31 | 9,4 | 26,6 | 30,8 | - |
| 32 | 0,0 | 1,9 | 12,4 | - |

TABLEAU 4

**Mesure de l'accumulation intracellulaire en adriamycine par les cellules de la lignée KB-A1**

| PRODUITS | VAR-MEAN AUX CONCENTRATIONS DE | | | |
|---|---|---|---|---|
| | 0,5 µM | 1,0 µM | 2,5 µM | 5,0 µM |
| PRODUIT DE REFERENCE VERAPAMIL | 6,4 | 8,0 | 11,1 | 17,0 |
| PRODUIT DES EXEMPLES | | | | |
| 1 | 4,7 | 4,5 | 8,2 | 11,2 |
| 2 | 3,6 | 5,6 | 16,2 | 26,2 |
| 3 | 22,5 | 27,5 | 40,9 | 47,6 |
| 4 | 13,0 | 17,9 | 25,8 | 34,2 |
| 5 | 20,1 | 25,9 | 34,5 | 42,0 |
| 6 | 3,1 | 4,8 | 18,2 | 29,2 |
| 7 | 1,9 | 1,0 | 1,9 | 1,9 |
| 8 | 0,4 | 0,4 | 6,7 | 9,8 |
| 9 | 24,9 | 31,2 | 37,3 | 43,9 |
| 10 | 8,2 | 11,7 | 17,2 | 22,2 |
| 11 | 35,1 | 38,6 | 49,3 | 54,1 |
| 12 | 4,1 | 4,2 | 8,4 | 14,5 |
| 13 | 11,3 | 12,9 | 16,2 | 20,6 |
| 14 | 7,9 | 11,3 | 12,8 | 16,4 |
| 15 | 3,4 | 4,9 | 11,5 | 20,9 |
| 16 | 2,6 | 2,5 | 6,4 | 13,0 |
| 17 | 2,6 | 3,1 | 4,2 | 5,5 |
| 18 | 0,0 | 2,0 | 8,0 | 16,3 |
| 19 | 23,4 | 29,7 | 40,0 | 47,6 |
| 20 | 22,0 | 21,6 | 30,8 | 44,1 |
| 21 | 13,5 | 15,1 | 26,9 | 39,8 |
| 22 | 18,9 | 23,2 | 35,8 | 45,9 |
| 23 | 8,5 | 13,8 | 22,1 | 29,8 |
| 24 | 5,0 | 10,0 | 15,9 | 22,8 |
| 25 | 7,6 | 9,4 | 16,4 | 29,7 |
| 26 | 22,8 | 29,6 | 42,5 | 57,3 |
| 27 | 10,2 | 25,3 | 37,2 | 48,4 |
| 28 | 24,7 | 27,0 | 38,5 | 35,9 |
| 29 | 24,9 | 33,2 | 37,2 | 38,8 |
| 30 | 11,3 | 14,8 | 16,7 | 25,6 |
| 31 | 32,8 | 44,7 | 57,4 | 72,6 |
| 32 | 14,9 | 23,8 | 36,5 | 49,4 |

TABLEAU 5

**Augmentation de l'activite antitumorale de la vincristine par les produits de la présente invention, et un produit de référence (Verapamil) sur la lignée résistante P/388/VCR**

| PRODUITS TESTES | DOSE mg/kg i.p. | T/C % |
|---|---|---|
| AUCUN PRODUIT | - | 146 ± 2 |
| PRODUIT DE REFERENCE | | |
| VERAPAMIL | 50 | 164 |
| | 75 | 177 |
| PRODUITS DES EXEMPLES | | |
| 3 | 50 | 208 ± 23 |
| 3 | 100 | 193 ± 3 |
| 5 | 50 | 184 |
| 5 | 100 | 195 |
| 11 | 50 | 156 |
| 11 | 100 | 168 ± 15 |
| 19 | 50 | 167 ± 7 |
| 19 | 100 | 184 ± 7 |
| 20 | 50 | 161 |
| 20 | 100 | 174 |
| 21 | 50 | 154 |
| 21 | 100 | 148 |
| 22 | 50 | 159 |
| 22 | 100 | 165 |
| 28 | 50 | 170 |
| 28 | 100 | 171 |
| 29 | 50 | 171 ± 6 |
| 29 | 100 | 185 ± 7 |
| 31 | 50 | 159 |
| 31 | 100 | 177 |

Les résultats de cette étude pharmacologique démontrent, dans leur ensemble, la supériorité des composés de la présente invention par rapport au Vérapamil pris comme produit de référence.

## Revendications

**Revendications pour les Etats contractants suivants : AT, BE, CH, DE, DK, FR, GB, IT, LI, LU, NL, PT, SE**

**1.** Les triazines et pyrimidines trisubstituées de formule générale I :

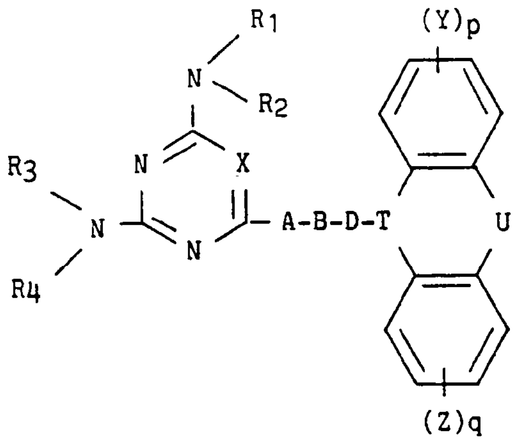

(I)

dans laquelle :

- $R_1$, $R_2$, $R_3$ et $R_4$ : identiques ou différents, représentent chacun : un atome d'hydrogène, un radical cycloalkyle ayant de 3 à 6 atomes de carbone ou un radical alkyle en chaine droite ou ramifiée, ayant de 1 à 6 atomes de carbone, renfermant éventuellement une double ou une triple liaison et éventuellement substitué par un atome d'halogène, un ou plusieurs radicaux hydroxy, ou un reste amino

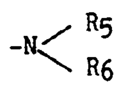 ,

dans lequel $R_5$ et $R_6$, identiques ou différents, représentent chacun un atome d'hydrogène ou un radical alkyle ayant de 1 à 5 atomes de carbone ou $R_6$ et $R_6$ forment ensemble avec l'atome d'azote auquel ils sont liés un hétérocycle penta- hexa- ou heptagonal renfermant éventuellement un atome d'oxygène ou de soufre ;
- X représente un atome d'azote ou le groupe CH ;
- A représente une liaison simple, un radical hydrocarboné de 1 à 3 atomes de carbone en chaine droite ou ramifiée, ou un groupement -NH-A'- dans lequel A' représente une chaine hydrocarbonée ayant de 2 à 6 atomes de carbone renfermant éventuellement un atome d'oxygène ou de soufre et éventuellement substituée par un radical hydroxy ;
- B représente un radical hétérocyclique de formule :

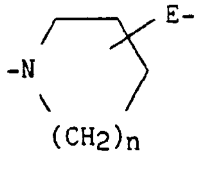

ou

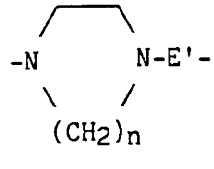

étant défini que :

. n représente un nombre entier de 1 à 3,

. E représente un atome d'oxygène ou de soufre ou un radical -NR- ou $-CH_2NR-$, R étant un atome d'hydrogène ou un radical alkyle ou alkényle ayant chacun jusqu'à 5 atomes de carbone, et

. E' représente une liaison simple ou un radical -NR- tel que défini ci-dessus ;

- D est une liaison simple ou un radical hydrocarboné, en chaine droite ou ramifiée, ayant jusqu'à 6 atomes de carbone ;
- T représente :

. un radical CR' dans lequel R' représente un atome d'hydrogène, un radical alkyle ayant de 1 à 5 atomes de carbone,

. un radical

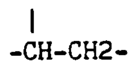

ou

. un atome d'azote ;

- U représente :
  . une liaison simple,
  . un radical CHR" dans lequel R" représente un atome d'hydrogène ou un radical alkyle ayant de 1 à 5 atomes de carbone,
  . un radical di- ou tri-méthylène [$(CH_2)_2$ et $(CH_2)_3$],
  . un radical -CH=CH,
  . un atome d'oxygène ou de soufre,
  . un radical -NR"' dans lequel R"' représente un atome d'hydrogène ou un radical alkyle ayant de 1 à 5 atomes de carbone,

. un radical de formule :

$CO$, $SO_2$, $-CH_2-O-$, $-CH_2-S-$, $-CH_2-SO_2-$,

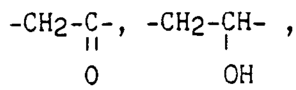

-CH=N-,

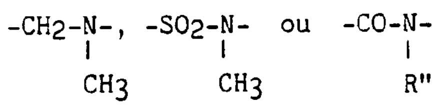

ou

dans lequel R"' est tel que précédemment défini ; et

- de plus lorsque T et U sont respectivement CR' et CHR" et R' et R" sont différents de H, R' et R" peuvent représenter ensemble un pont polyméthylénique ayant 2 ou 3 atomes de carbone ;
- Y et Z, identiques ou différents représentent chacun un atome d'hydrogène, un atome d'halogène, un radical trifluorométhyle ou un radical alkyle ou alkoxy ayant chacun de 1 à 3 atomes de carbone ; et
- p et q, identiques ou différents prennent chacun les valeurs 1 ou 2 ;

et, lorsque la formule générale I renferme un ou plusieurs atomes de carbone chiraux, les énantiomères ou diastéréoisomères correspondants.

2. Les sels physiologiquement tolérables des composés de la revendication 1 avec des acides appropriés.

3. La 2,4-diallylamino-6-{4-[(10,11-dihydro-5H-dibenzo (a,d) cycloheptén-5-yl) méthylamino] pipéridin-1-yl}-1,3,5-triazine.

4. La 2,4-diallylamino-6-[4-(10,11-dihydro-5H-dibenzo (a,d) cycloheptén-5-yl amino) pipéridin-1-yl]-1,3,5-triazine, et son fumarate.

5. La 2,4-diallylamino-6-{4-[(5H-dibenzo (a,d) cycloheptén-5-yl) méthylamino] pipéridin-1-yl}-1,3,5-triazine et son difumarate.

6. La 2,4-diallylamino-6-{4-[(R,S) 6,11-dihydro-11H-dibenzo (b,e) oxépin-11-yl) méthylamino] pipéridin-1-yl}-1,3,5-triazine.

7. La 2,4-diallylamino-6-{4-[(8-chloro-10,10-dioxo-11-méthyl (R,S) dibenzo (c,f) thiazépin-5-yl) méthylamino] pipéridin-1-yl}-1,3,5-triazine.

8. La 2-allylamino-4-éthylamino-6-{4-[(10,11-dihydro-5H-dibenzo (a,d) cycloheptén-5-yl) méthylamino] pipéridin-1-yl}-1,3,5-triazine.

9. La 2-allylamino-4-propylamino-6-{4-[(10,11-dihydro-5H-dibenzo (a,d) cycloheptén-5-yl) méthylamino] pipéridin-1-yl}-1,3,5-triazine.

10. Le procédé de préparation des composés de la revendication 1, caractérisé en ce que l'on condense :

- soit un composé de formule générale II :

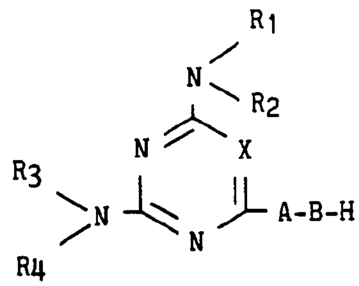

(II)

dans laquelle $R_1$, $R_2$, $R_3$, $R_4$, X, A et B ont les significations définies dans la revendication 1, avec un composé de formule générale III :

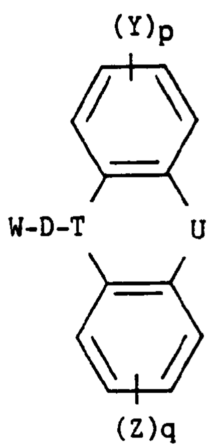

(III)

dans laquelle D, T, U, Y, Z, p et q ont les significations définies dans la revendication 1, et W représente un atome d'halogène tel que par exemple un atome de chlore ou de brome, ou un reste tosyloxy ;

- soit un composé halogéné de formule générale IV :

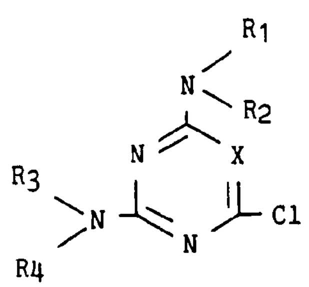

(IV)

dans laquelle $R_1$, $R_2$, $R_3$, $R_4$ et X ont les significations définies dans la revendication 1, avec un composé de formule générale V :

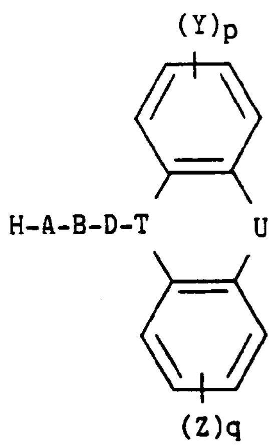

(V)

dans laquelle A, B, D, T, U, Y, Z, p et q ont les significations définies dans la revendication 1.

**11.** Le procédé de préparation des composés de la revendication 1 répondant à la formule générale I' :

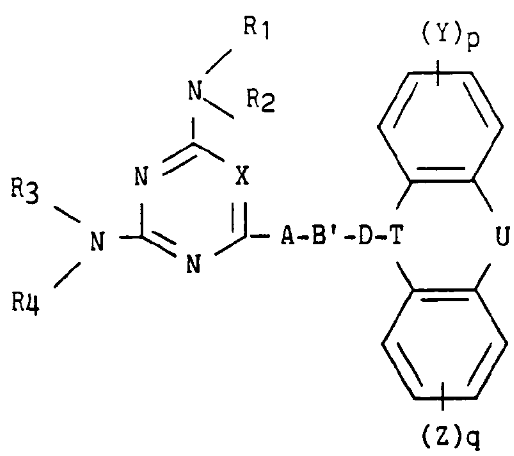

(I')

dans laquelle :

- $R_1$, $R_2$, $R_3$, $R_4$, X, A, D, T, U, Y, Z, p, et q ont les significations définies dans la revendication 1, et
- B' représente le radical :

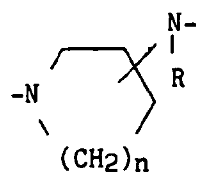

dans lequel n et R ont les significations définies dans la revendication 1, caractérisé en ce que l'on condense

. un composé de formule générale VI :

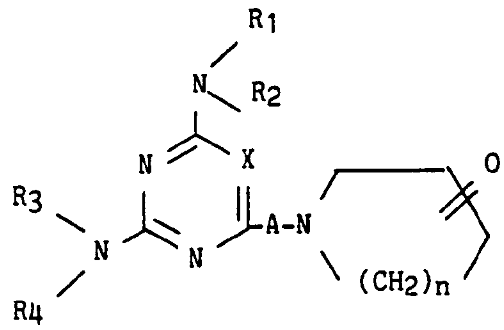

(VI)

dans laquelle $R_1$, $R_2$, $R_3$, $R_4$, X, A et n ont les significations défines dans la revendication 1,

. avec un composé de formule générale VII :

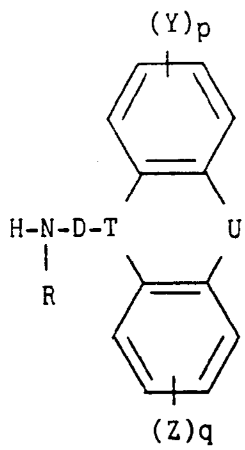

(VII)

dans laquelle R, D, T, U, Y, Z, p et q ont les significations définies dans la revendication 1.

**12.** Les compositions pharmaceutiques contenant comme principe actif un composé selon les revendications 1 à 9, avec des excipients pharmaceutiques appropriés.

**13.** Les compositions pharmaceutiques selon la revendication 12 présentées sous une forme convenant notamment pour supprimer la résistance des cellules tumorales aux agents anti-cancéreux et pour supprimer la résistance des parasites aux agents anti-parasitaires.

**Revendications pour les Etats contractants suivants : ES, GR**

**1.** Procédé de préparation des triazines et pyrimidines trisubstituées de formule générale I :

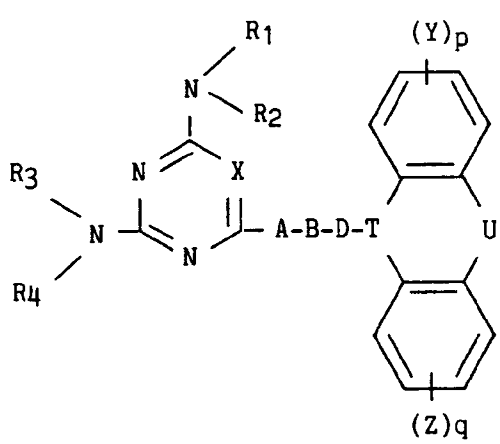

(I)

dans laquelle :

- $R_1$, $R_2$, $R_3$ et $R_4$ : identiques ou différents, représentent chacun : un atome d'hydrogène, un radical cycloalkyle ayant de 3 à 6 atomes de carbone ou un radical alkyle en chaine droite ou ramifiée, ayant de 1 à 6 atomes de carbone, renfermant éventuellement une double ou une triple liaison et éventuellement substitué par un atome d'halogène, un ou plusieurs radicaux hydroxy, ou un reste amino

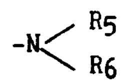 ,

dans lequel $R_5$ et $R_6$, identiques ou différents, représentent chacun un atome d'hydrogène ou un radical alkyle ayant de 1 à 5 atomes de carbone ou $R_6$ et $R_6$ forment ensemble avec l'atome d'azote auquel ils sont liés un hétérocycle penta- hexa- ou heptagonal renfermant éventuellement un atome d'oxygène ou de soufre ;
- X représente un atome d'azote ou le groupe CH ;
- A représente une liaison simple, un radical hydrocarboné de 1 à 3 atomes de carbone en chaine droite ou ramifiée, ou un groupement -NH-A'- dans lequel A' représente une chaine hydrocarbonée ayant de 2 à 6 atomes de carbone renfermant éventuellement un atome d'oxygène ou de soufre et éventuellement substituée par un radical hydroxy ;
- B représente un radical hétérocyclique de formule :

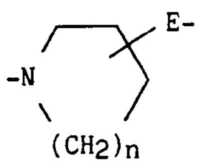

ou

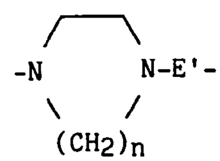

étant défini que :

. n représente un nombre entier de 1 à 3,

. E représente un atome d'oxygène ou de soufre ou un radical -NR- ou $-CH_2NR-$, R étant un atome d'hydrogène ou un radical alkyle ou alkényle ayant chacun jusqu'à 5 atomes de carbone, et

. E' représente une liaison simple ou un radical -NR- tel que défini ci-dessus ;

- D est une liaison simple ou un radical hydrocarboné, en chaine droite ou ramifiée, ayant jusqu'à 6 atomes de carbone ;
- T représente :

. un radical CR' dans lequel R' représente un atome d'hydrogène, un radical alkyle ayant de 1 à 5 atomes de carbone,

. un radical

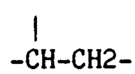

ou

. un atome d'azote ;

- U représente :

. une liaison simple,

. un radical CHR" dans lequel R" représente un atome d'hydrogène ou un radical alkyle ayant de 1 à 5 atomes de carbone,

. un radical di- ou tri-méthylène [$(CH_2)_2$ et $(CH_2)_3$],

. un radical -CH=CH,

. un atome d'oxygène ou de soufre,

. un radical -NR"' dans lequel R"' représente un atome d'hydrogène ou un radical alkyle ayant de 1 à 5 atomes de carbone,

. un radical de formule :

CO, $SO_2$, $-CH_2-O-$, $-CH_2-S-$, $-CH_2-SO_2-$,

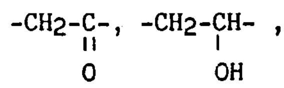

-CH=N-,

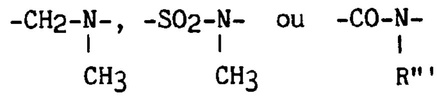

dans lequel R"' est tel que précédemment défini ; et

- de plus lorsque T et U sont respectivement CR' et CHR" et R' et R" sont différents de H, R' et R" peuvent représenter ensemble un pont polyméthylénique ayant 2 ou 3 atomes de carbone ;
- Y et Z, identiques ou différents représentent chacun un atome d'hydrogène, un atome d'halogène, un radical trifluorométhyle ou un radical alkyle ou alkoxy ayant chacun de 1 à 3 atomes de carbone ; et
- p et q, identiques ou différents prennent chacun les valeurs 1 ou 2 ;

et, lorsque la formule générale I renferme un ou plusieurs atomes de carbone chiraux, les énantiomères ou diastéréoisomères correspondants,
et leurs sels physiologiquement tolérables avec des acides appropriés,
caractérisé en ce que l'on condense :

- soit un composé de formule générale II :

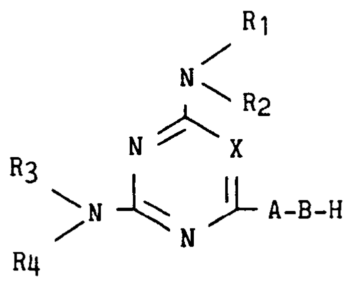

(II)

dans laquelle $R_1$, $R_2$, $R_3$, $R_4$, X, A et B ont les significations précédemment définies, avec un composé de formule générale III :

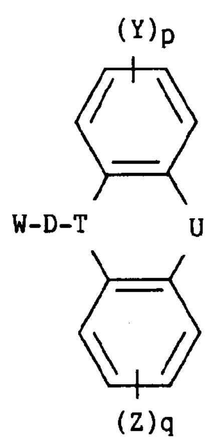

(III)

dans laquelle D, T, U, Y, Z, p et q ont les significations précédemment définies, et W représente un atome d'halogène ou un reste tosyloxy ;

- soit un composé halogéné de formule générale IV :

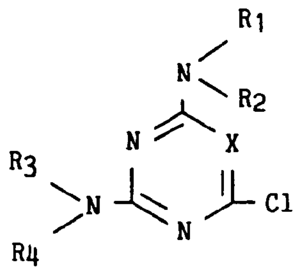

(IV)

dans laquelle $R_1$, $R_2$, $R_3$ , $R_4$ et X ont les significations précédemment définies, avec un composé de formule générale V :

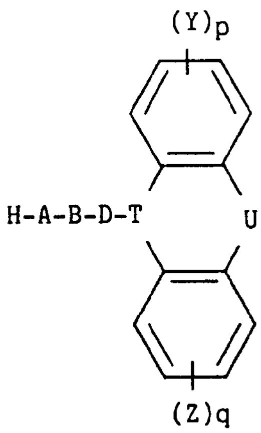

(V)

dans laquelle A, B, D, T, U, Y, Z, p et q ont les significations précédemment définies, et si on le désire, les composés de formule I ainsi obtenus sont traités avec des acides physiologiquement tolérables pour donner les sels d'addition acides correspondants.

**2.** Le procédé de préparation des composés de formule I définie dans la revendication 1 répondant à la formule générale I' :

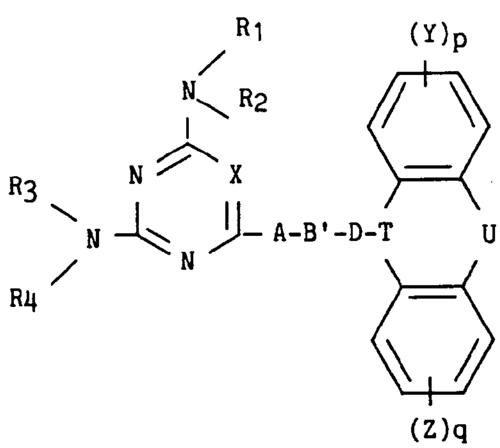

(I')

dans laquelle :

- $R_1$, $R_2$, $R_3$, $R_4$, X, A, D, T, U, Y, Z, p, et q ont les significations définies dans la revendication 1, et
- B' représente le radical :

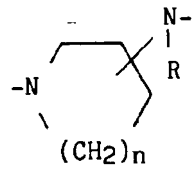

dans lequel n et R ont les significations définies dans la revendication 1, caractérisé en ce que l'on condense

. un composé de formule générale VI :

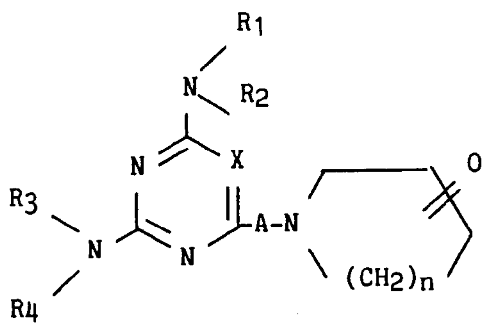

(VI)

dans laquelle $R_1$, $R_2$, $R_3$, $R_4$, X, A et n ont les significations défines dans la revendication 1,

. avec un composé de formule générale VII :

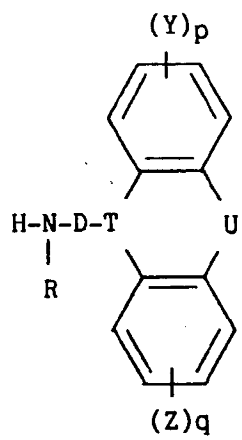

(VII)

dans laquelle R, D, T, U, Y, Z, p et q ont les significations définies précédemment ;

et si on le désire, les composés I' ainsi obtenus sont traités avec des acides physiologiquement tolérables pour donner les sels d'addition acides correspondants.

## Patentansprüche

### Patentansprüche für folgende Vertragsstaaten : AT, BE, CH, LI, DE, DK, FR, GB, IT, LU, NL, PT, SE

1. Trisubstituierte Triazine und Pyrimidine der allgemeinen Formel I:

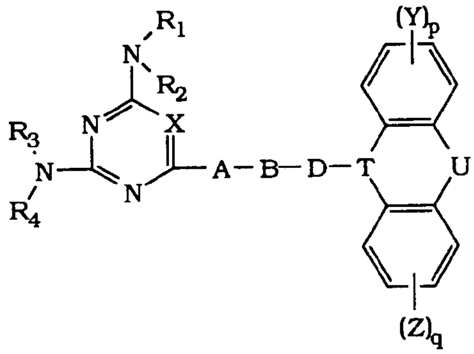

(I)

in der:

- $R_1$, $R_2$, $R_3$ und $R_4$, die gleichartig oder verschieden sein können, jeweils ein Wasserstoffatom, eine Cycloalkylgruppe mit 3 bis 6 Kohlenstoffatomen oder eine Alkylgruppe mit gerader oder verzweigter Kette und 1 bis 6 Kohlenstoffatomen, die gegebenenfalls eine Doppelbindung oder eine Dreifachbindung umfassen kann und gegebenenfalls durch ein Halogenatom oder einen oder mehrere Hydroxylgruppen oder eine Aminogruppe

  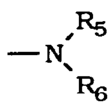

  in der $R_5$ und $R_6$ die gleichartig oder verschieden sein können, jeweils ein Wasserstoffatom oder eine Alkylgruppe mit 1 bis 5 Kohlenstoffatomen darstellen oder $R_5$ und $R_5$ gemeinsam mit dem Stickstoffatom, an das sie gebunden sind, einen penta-, hexa- oder heptagonalen Heterocyclus bilden, der gegebenenfalls ein Sauerstoffatom oder ein Schwefelatom enthält, substituiert sein kann;
- X ein Stickstoffatom oder die Gruppe CH;
- A eine Einfachbindung, eine Kohlenwasserstoffgruppe mit 1 bis 3 Kohlenstoffatomen mit gerader oder verzweigter Kette oder eine Gruppe -NH-A'-, in der A' eine Kohlenwasserstoffkette mit 2 bis 6 Kohlenstoffatomen darstellt, die gegebenenfalls ein Sauerstoffatom oder ein Schwefelatom umfaßt und gegebenenfalls durch eine Hydroxylgruppe substituiert ist;
- B eine heterocyclische Gruppe der Formel

  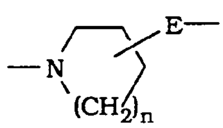 oder 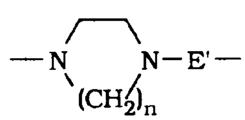

  wobei:
  . n eine ganze Zahl mit einem Wert von 1 bis 3,
  . E ein Sauerstoffatom oder ein Schwefelatom oder eine Gruppe -NR- oder $-CH_2NR-$, worin R ein Wasserstoffatom oder eine Alkylgruppe oder Alkenylgruppe mit jeweils bis zu 5 Kohlenstoffatomen darstellt, und
  . E' eine Einfachbindung oder eine Gruppe -NR-, wie sie oben definiert worden ist, bedeuten;
- D eine Einfachbindung oder eine Kohlenwasserstoffgruppe mit gerader oder verzweigter Kette, die bis zu 6 Kohlenstoffatome umfaßt;
- T
  . eine Gruppe CR', in der R' ein Wasserstoffatom oder eine Alkylgruppe mit 1 bis 5 Kohlenstoffatomen darstellt,
  . eine Gruppe

  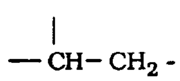

oder

. ein Stickstoffatom darstellt;

- U

. eine Einfachbindung,

. eine Gruppe CHR", worin R" ein Wasserstoffatom oder eine Alkylgruppe mit 1 bis 5 Kohlenstoffatomen darstellt,

. eine Di- oder Tri-methylengruppe [$(CH_2)_2$ und $(CH_2)_3$],

. eine Gruppe -CH=CH,

. ein Sauerstoffatom oder ein Schwefelatom,

. eine Gruppe NR"', worin R"' ein Wasserstoffatom oder eine Alkylgruppe mit 1 bis 5 Kohlenstoffatomen darstellt,

. eine Gruppe der Formel

CO, $SO_2$, $-CH_2-O-$, $-CH_2-S-$, $-CH_2-SO_2-$,

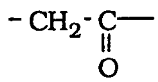,
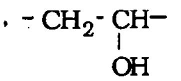,

-CH=N-,

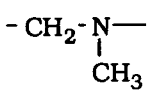,
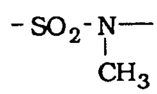
oder
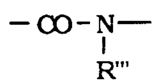

worin R"' die oben angegebenen Bedeutungen besitzt;

- mit der Maßgabe, daß wenn T und U CR' beziehungsweise CHR" bedeuten, R' und R" von H verschieden sind, wobei R' und R" gemeinsam eine Polymethylenbrücke mit 2 oder 3 Kohlenstoffatomen darstellen können; und
- Y und Z, die gleichartig oder verschieden sein können, jeweils ein Wasserstoffatom, ein Halogenatom. eine Trifluormethylgruppe oder eine Alkylgruppe oder Alkoxygruppe mit jeweils 1 bis 3 Kohlenstoffatomen bedeuten; und
- p und q, die gleichartig oder verschieden sein können, jeweils die Werte 1 oder 2 besitzen;

und, wenn die allgemeine Formel I eines oder mehrere chirale Kohlenstoffatome umfaßt, die entsprechenden Enantiomere oder Diastereoisomere.

**2.** Die physiologisch verträglichen Salze der Verbindungen nach Anspruch 1 mit geeigneten Säuren.

**3.** 2,4-Diallylamino-6-{4-[(10,11-dihydro-5H-dibenzo(a,d)cyclohepten-5-yl)-methylamino]-piperidin-1-yl}-1,3,5-triazin.

**4.** 2,4-Diallylamino-6-[4-(10,11-dihydro-5H-dibenzo(a,d)cyclohepten-5-yl-amino)-piperidin-1-yl]-1,3,5-triazin und dessen Fumarat.

**5.** 2,4-Diallylamino-6-{4-[(5H-dibenzo(a,d)cyclohepten-5-yl)-methylamino]-piperidin-1-yl}-1,3,5-triazin und dessen Difumarat.

**6.** 2,4-Diallylamino-6-{4-[(R,S)-6,11-dihydro-11H-dibenzo(b,e)oxepin-11-yl)-methylamino]-piperidin-1-yl}-1,3,5-triazin.

**7.** 2,4-Diallylamino-6-{4-[8-chlor-10,10-dioxo-11-methyl(R,S)dibenzo(c,f)thiazepin-5-yl)-methylamino]-piperidin-1-yl}-1,3,5-triazin.

**8.** 2-Allylamino-4-ethylamino-6-{4-[10,11-dihydro-5H-dibenzo(a,d)cyclohepten-5-yl)-methylamino]-piperidin-1-yl}-1,3,5-triazin.

**9.** 2-Allylamino-4-propylamino-6-{4-[(10,11-dihydro-5H-dibenzo(a,d)cyclohepten-5-yl)-methylamino]-piperidin-1-yl}-1,3,5-triazin.

**10.** Verfahren zur Herstellung der Verbindung nach Anspruch 1, **dadurch gekennzeichnet,** daß man

- entweder eine Verbindung der allgemeinen Formel II

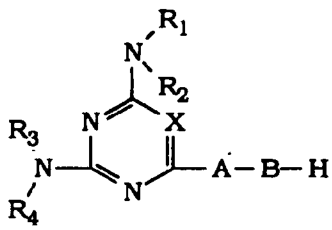

(II)

in der $R_1$, $R_2$, $R_3$, $R_4$, X, A und B die in Anspruch 1 angegebenen Bedeutungen besitzen, mit einer Verbindung der allgemeinen Formel III

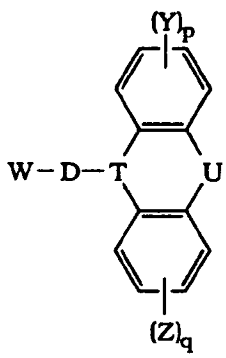

(III)

in der D, T, U, Y, Z, p und q die in Anspruch 1 angegebenen Bedeutungen besitzen und W ein Halogenatom, wie beispielsweise ein Chloratom, oder einen Toxyloxyrest darstellt, kondensiert;

- oder eine Halogenverbindung der allgemeinen Formel IV

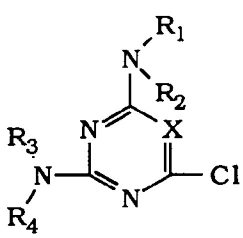

(IV)

in der $R_1$, $R_2$, $R_3$, $R_4$ und X die in Anspruch 1 angegebenen Bedeutungen besitzen, mit einer Verbindung der allgemeinen Formel V:

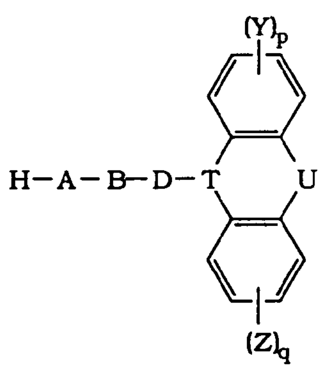

(V)

in der A, B, D, T, U, Y, Z, p und q die in Anspruch 1 angegebenen Bedeutungen besitzen, kondensiert.

**11.** Verfahren zur Herstellung von Verbindungen nach Anspruch 1 der allgemeinen Formel I'

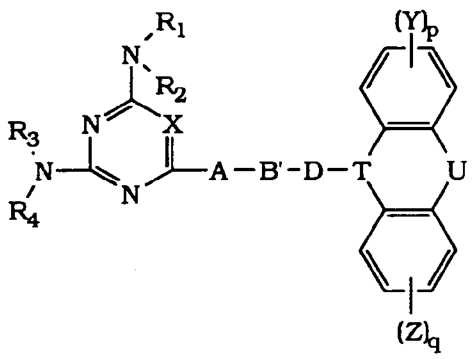

(I')

in der
- $R_1$, $R_2$, $R_3$, $R_4$, X, A, D, T, U, Y, Z, p und q die in Anspruch 1 angegebenen Bedeutungen besitzen, und
- B' die Gruppe

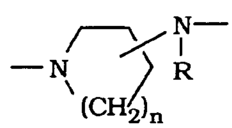

bedeutet, in der n und R die in Anspruch 1 angegebenen Bedeutungen besitzen, **dadurch gekennzeichnet**, daß man

. eine Verbindung der allgemeinen Formel VI

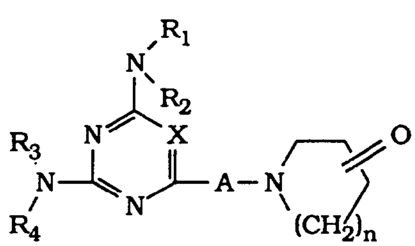

(VI)

in der $R_1$, $R_2$, $R_3$, $R_4$, X, A und n die in Anspruch 1 angegebenen Bedeutungen besitzen,

. mit einer Verbindung der allgemeinen Formel VII

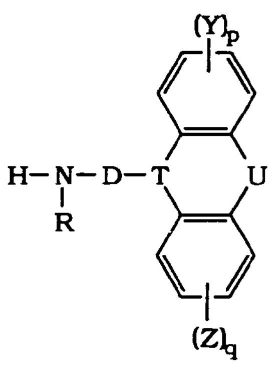

(VII)

in der R, D, T, U, Y, Z, p und q die in Anspruch 1 angegebenen Bedeutungen besitzen, kondensiert.

**12.** Pharmazeutische Zubereitungen enthaltend als Wirkstoff eine Verbindung nach den Ansprüchen 1 bis 9,

zusammen mit geeigneten pharmazeutischen Trägermaterialien.

13. Pharmazeutische Zubereitungen nach Anspruch 12 in einer Form, die insbesondere in einer zur Unterdrückung der Resistenz von Tumorzellen gegen Antikrebsmittel und zur Unterdrückung der Resistenz von Parasiten gegen Anti-Parasitenmittel geeigneten Form vorliegen.

**Patentansprüche für folgende Vertragsstaaten : ES, GR**

1. Verfahren zur Herstellung von trisubstituierten Triazinen und Pyrimidinen der allgemeinen Formel I

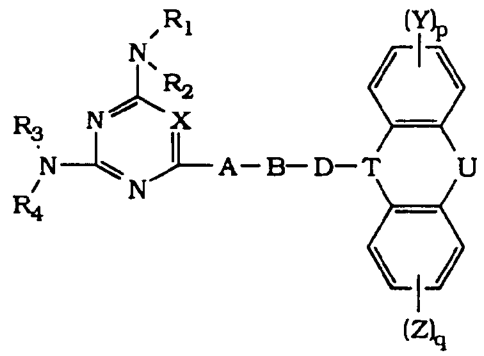

(I)

in der:

- $R_1$, $R_2$, $R_3$ und $R_4$, die gleichartig oder verschieden sein können, jeweils ein Wasserstoffatom, eine Cycloalkylgruppe mit 3 bis 6 Kohlenstoffatomen oder eine Alkylgruppe mit gerader oder verzweigter Kette und 1 bis 6 Kohlenstoffatomen, die gegebenenfalls eine Doppelbindung oder eine Dreifachbindung umfassen kann und gegebenenfalls durch ein Halogenatom oder einen oder mehrere Hydroxyl- gruppen oder eine Aminogruppe

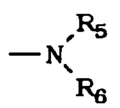

  in der $R_5$ und $R_6$ die gleichartig oder verschieden sein können, jeweils ein Wasserstoffatom oder eine Alkylgruppe mit 1 bis 5 Kohlenstoffatomen darstellen oder $R_5$ und $R_5$ gemeinsam mit dem Stickstoffatom, an das sie gebunden sind, einen penta-, hexa- oder heptagonalen Heterocyclus bilden, der gegebenenfalls ein Sauerstoffatom oder ein Schwefelatom enthält, substituiert sein kann;
- X ein Stickstoffatom oder die Gruppe CH;
- A eine Einfachbindung, eine Kohlenwasserstoffgruppe mit 1 bis 3 Kohlenstoffatomen mit gerader oder verzweigter Kette oder eine Gruppe -NH-A'-, in der A' eine Kohlenwasserstoffkette mit 2 bis 6 Kohlenstoffatomen darstellt, die gegebenenfalls ein Sauerstoffatom oder ein Schwefelatom umfaßt und gegebenenfalls durch eine Hydroxylgruppe substituiert ist;
- B eine heterocyclische Gruppe der Formel

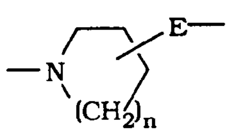

oder

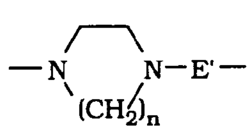

  wobei:
  . n eine ganze Zahl mit einem Wert von 1 bis 3,
  . E ein Sauerstoffatom oder ein Schwefelatom oder eine Gruppe -NR- oder $-CH_2NR-$, worin R ein Wasserstoffatom oder eine Alkylgruppe oder Alkenylgruppe mit jeweils bis zu 5 Kohlenstoffatomen darstellt, und
  . E' eine Einfachbindung oder eine Gruppe -NR-, wie sie oben definiert worden ist, bedeuten;

- D eine Einfachbindung oder eine Kohlenwasserstoffgruppe mit gerader oder verzweigter Kette, die bis zu 6 Kohlenstoffatome umfaßt;
- T
  . eine Gruppe CR', in der R' ein Wasserstoffatom oder eine Alkylgruppe mit 1 bis 5 Kohlenstoffatomen darstellt,
  . eine Gruppe

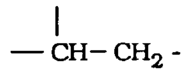

  oder
  . ein Stickstoffatom bedeuten;
- U
  . eine Einfachbindung,
  . eine Gruppe CHR", worin R" ein Wasserstoffatom oder eine Alkylgruppe mit 1 bis 5 Kohlenstoffatomen darstellt,
  . eine Di- oder Tri-methylengruppe [$(CH_2)_2$ und $(CH_2)_3$],
  . eine Gruppe -CH=CH,
  . ein Sauerstoffatom oder ein Schwefelatom,
  . eine Gruppe NR"', worin R"' ein Wasserstoffatom oder eine Alkylgruppe mit 1 bis 5 Kohlenstoffatomen darstellt,
  . eine Gruppe der Formel
  CO, $SO_2$, $-CH_2O-$, $-CH_2-S-$, $-CH_2-SO_2-$,

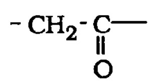 , 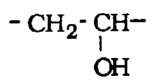 ,

  -CH=N-,

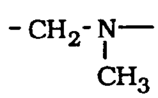 , 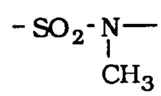 oder 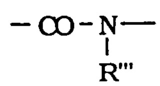

  worin R"' die oben angegebenen Bedeutungen besitzt;
- mit der Maßgabe, daß wenn T und U CR' beziehungsweise CHR" bedeuten, R' und R" von H verschieden sind, wobei R' und R" gemeinsam eine Polymethylenbrücke mit 2 oder 3 Kohlenstoffatomen darstellen können; und
- Y und Z, die gleichartig oder verschieden sein können, jeweils ein Wasserstoffatom, ein Halogenatom, eine Trifluormethylgruppe oder eine Alkylgruppe oder Alkoxygruppe mit jeweils 1 bis 3 Kohlenstoffatomen bedeuten; und
- p und q, die gleichartig oder verschieden sein können, jeweils die Werte 1 oder 2 besitzen;

und, wenn die allgemeine Formel I eines oder mehrere chirale Kohlenstoffatome umfaßt, von entsprechenden Enantiomeren oder Diastereoisomeren; und von deren physiologisch verträglichen Salzen mit geeigneten Säuren, **dadurch gekennzeichnet**, daß man

- entweder eine Verbindung der allgemeinen Formel II

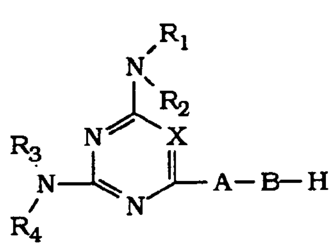

(II)

in der $R_1$, $R_2$, $R_3$, $R_4$, X, A und B die bereits oben angegebenen Bedeutungen besitzen, mit einer Verbindung der allgemeinen Formel III

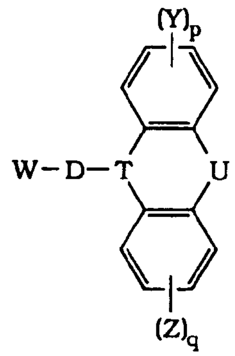

(III)

in der D, T, U, Y, Z, p und q die bereits oben angegebenen Bedeutungen besitzen und W ein Halogenatom,

. oder einen Toxyloxyrest darstellt, kondensiert;

- oder eine Halogenverbindung der allgemeinen Formel IV

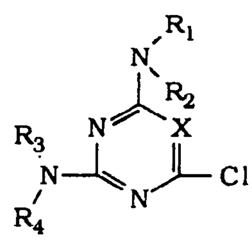

(IV)

in der $R_1$, $R_2$, $R_3$, $R_4$ und X die bereits oben angegebenen Bedeutungen besitzen, mit einer Verbindung der allgemeinen Formel V:

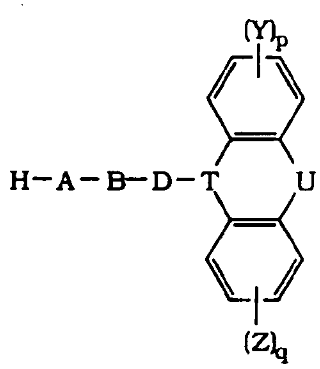

(V)

in der A, B, D, T, U, Y, Z, p und q die bereits oben angegebenen Bedeutungen besitzen, kondensiert.

**2.** Verfahren zur Herstellung von Verbindungen nach Anspruch 1 der allgemeinen Formel I'

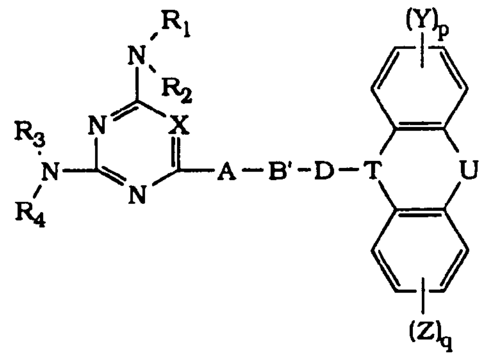

(I')

in der

- $R_1$, $R_2$, $R_3$, $R_4$, X, A, D, T, U, Y, Z, p und q die in Anspruch 1 angegebenen Bedeutungen besitzen, und
- B' die Gruppe

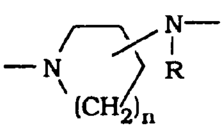

bedeutet, in der n und R die in Anspruch 1 angegebenen Bedeutungen besitzen, **dadurch gekennzeichnet,** daß man

. eine Verbindung der allgemeinen Formel VI

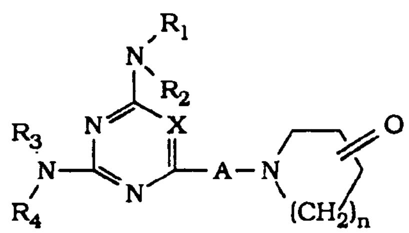

(VI)

in der $R_1$, $R_2$, $R_3$, $R_4$, X, A und n die in Anspruch 1 angegebenen Bedeutungen besitzen,

. mit einer Verbindung der allgemeinen Formel VII

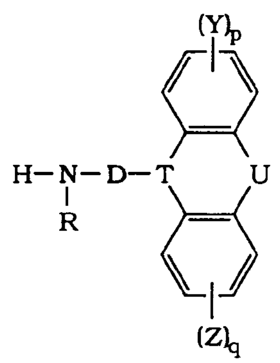

(VII)

in der R, D, T, U, Y, Z, p und q die in Anspruch 1 angegebenen Bedeutungen besitzen; kondensiert

und gewünschtenfalls die in dieser Weise erhaltenen Verbindungen 1' mit physiologisch verträglichen Säuren behandelt, um die entsprechenden Säureadditionssalze zu bilden.

## Claims

**Claims for the following Contracting States : AT, BE, CH, DE, DK, FR, GB, IT, LI, LU, NL, PT, SE**

**1.** Trisubstituted triazines and pyrimidines of the general formula I :

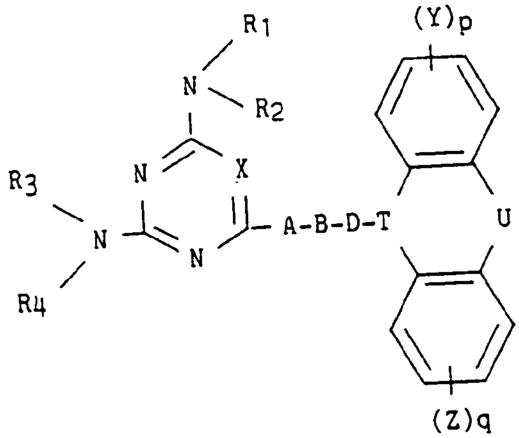

(I)

wherein :

- $R_1$, $R_2$, $R_3$ and $R_4$ are the same or different and each represents a hydrogen atom, a cycloalkyl radical having from 3 to 6 carbon atoms or a straight-chain or branched alkyl radical having from 1 to 6 carbon atoms, that optionally contains a double or a triple bond and is optionally substituted by a halogen atom, by one or more hydroxy radicals, or by an amino radical

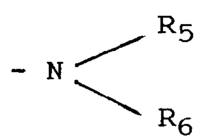

wherein $R_5$ and $R_6$ are the same or different and each represents a hydrogen atom or an alkyl radical having from 1 to 5 carbon atoms, or $R_5$ and $R_5$, together with the nitrogen atom to which they are bonded, form a pentagonal, hexagonal or heptagonal heterocycle optionally containing an oxygen or sulphur atom;
- X represents a nitrogen atom or the group CH;
- A represents a single bond, a straight-chain or branched hydrocarbon radical having from 1 to 3 carbon atoms, or a group -NH-A'- wherein A' represents a hydrocarbon chain having from 2 to 6 carbon atoms that optionally contains an oxygen or sulphur atom and is optionally substituted by a hydroxy radical;
- B represents a heterocyclic radical of formula :

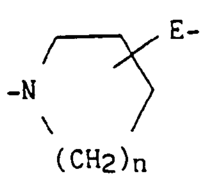

or

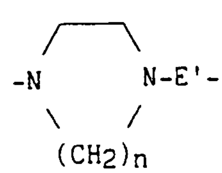

wherein :

. n represents an integer of from 1 to 3,

. E represents an oxygen or sulphur atom or an -NR- or $-CH_2NR-$ radical, R representing a hydrogen atom or an alkyl or alkenyl radical each having up to 5 carbon atoms, and

. E' represents a single bond or an -NR- radical as defined above;

- D represents a single bond or a straight-chain or branched hydrocarbon radical having up to 6 carbon atoms;
- T represents :
  . a CR' radical wherein R' represents a hydrogen atom or an alkyl radical having from 1 to 5 carbon atoms,
  . a

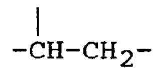

  radical or
  . a nitrogen atom;
- U represents :
  . a single bond,
  . a CHR" radical wherein R" represents a hydrogen atom or an alkyl radical having from 1 to 5 carbon atoms,
  . a di- or tri-methylene radical [$(CH_2)_2$ and $(CH_2)_3$],
  . a -CH=CH radical,
  . an oxygen or sulphur atom,
  . an -NR''' radical wherein R''' represents a hydrogen atom or an alkyl radical having from 1 to 5 carbon atoms,
  . a radical of formula :
  CO, $SO_2$, $-CH_2-O-$, $-CH_2-S-$, $-CH_2-SO_2-$,

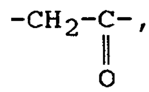

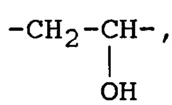 , $-CH=N-$ , 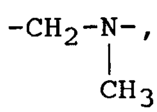 , 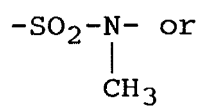 or 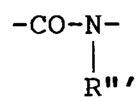

  wherein R''' is as defined hereinbefore; and
- furthermore, when T and U represent CR' and CHR" respectively and R' and R" are other than H, R' and R" may together represent a polymethylene bridge having 2 or 3 carbon atoms;
- Y and Z are the same or different and each represents a hydrogen atom, a halogen atom, a trifluoromethyl radical, or an alkyl or alkoxy radical each having from 1 to 3 carbon atoms;
  and
- p and q are the same or different and are each 1 or 2;

and, when the general formula I contains one or more chiral carbon atoms, the corresponding enantiomers or diastereoisomers.

2. The physiologically tolerable salts of compounds of claim 1 with appropriate acids.

3. 2,4-Diallylamino-6-{4-[(10,11-dihydro-5H-dibenzo[a,d]cyclohepten-5-yl)methylamino]piperidin-1-yl}-1,3,5-triazine.

4. 2,4-Diallylamino-6-[4-(10,11-dihydro-5H-dibenzo[a,d]cyclohepten-5-ylamino)piperidin-1-yl]-1,3,5-triazine and its fumarate.

5. 2,4-Diallylamino-6-{4-[(5H-dibenzo[a,d]cyclohepten-5-yl)methylamino]piperidin-1-yl}-1,3,5-triazine and its difumarate.

6. 2,4-Diallylamino-6-{4-[(R,S)-6,11-dihydro-11H-dibenzo[b,e]oxepin-11-yl)methylamino]piperidin-1-yl}-1,3,5-triazine.

7. 2,4-Diallylamino-6-{4-[(8-chloro-10,10-dioxo-11-methyl-(R,S)-dibenzo[c,f]thiazepin-5-yl)methylamino]piperidin-1-yl}-1,3,5-triazine.

8. 2-Allylamino-4-ethylamino-6-{4-[(10,11-dihydro-5H-dibenzo[a,d]cyclohepten-5-yl)methylamino]-piperidin-1-yl}-1,3,5-triazine.

9. 2-Allylamino-4-propylamino-6-{4-[(10,11-dihydro-5H-dibenzo[a,d]cyclohepten-5-yl)methylamino]-piperidin-1-yl}-1,3,5-triazine.

10. A process for the preparation of compounds of claim 1, characterised in that :
- either a compound of the general formula II :

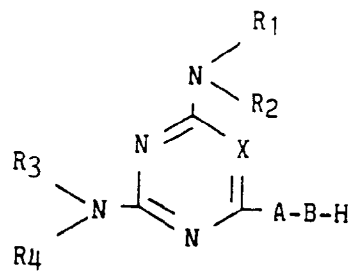

(II)

wherein $R_1$, $R_2$, $R_3$, $R_4$, X, A and B are as defined in claim 1, is condensed with a compound of the general formula III :

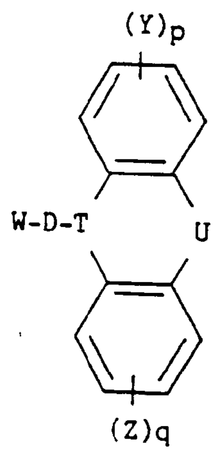

(III)

wherein D, T, U, Y, Z, p and q are as defined in claim 1 and W represents a halogen atom, such as, for example, a chlorine or bromine atom, or a tosyloxy radical;
- or a halogenated compound of the general formula IV :

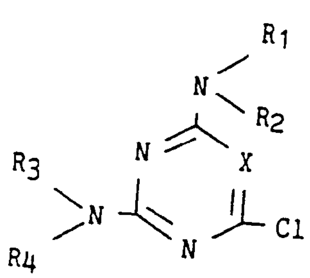

(IV)

wherein $R_1$, $R_2$, $R_3$, $R_4$ and X are as defined in claim 1, is condensed with a compound of the general formula V :

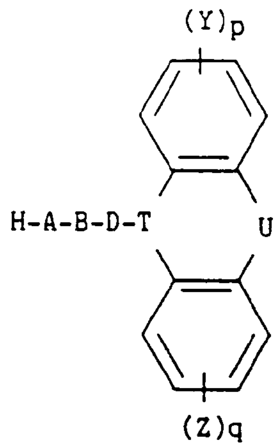

(V)

wherein A, B, D, T, U, Y, Z, p and q are as defined in claim 1.

**11.** A process for the preparation of compounds of claim 1 corresponding to the general formula I' :

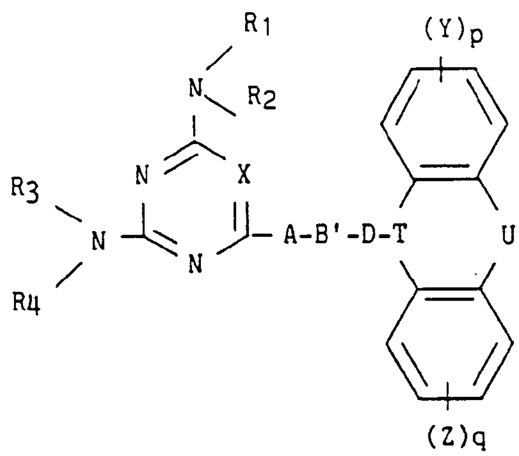

(I')

wherein :

- $R_1$, $R_2$, $R_3$, $R_4$, X, A, D, T, U, Y, Z, p and q are as defined in claim 1, and
- B' represents the radical :

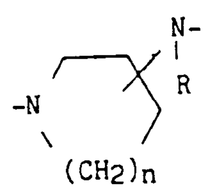

wherein n and R are as defined in claim 1, characterised in that

. a compound of the general formula VI :

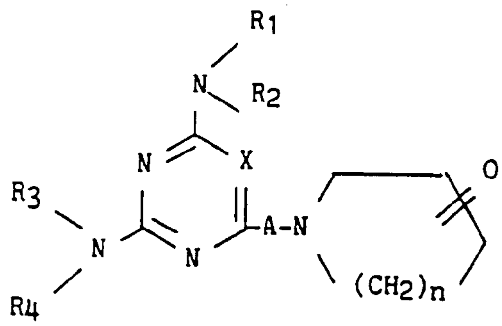

(VI)

wherein $R_1$, $R_2$, $R_3$, $R_4$, X, A and n are as defined in claim 1, is condensed with a compound of the general formula VII :

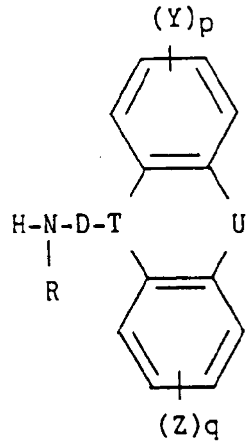

(VII)

wherein R, D, T, U, Y, Z, p and q are as defined in claim 1.

12. Pharmaceutical compositions comprising as active ingredient a compound according to claims 1 to 9 together with appropriate pharmaceutical excipients.

13. Pharmaceutical compositions according to claim 12 presented in a form suitable especially for suppressing the resistance of tumour cells to anti-cancer agents and for suppressing the resistance of parasites to anti-parasitic agents.

**Claims for the following Contracting States : ES GR**

1. A process for the preparation of trisubstituted triazines and pyrimidines of the general formula I :

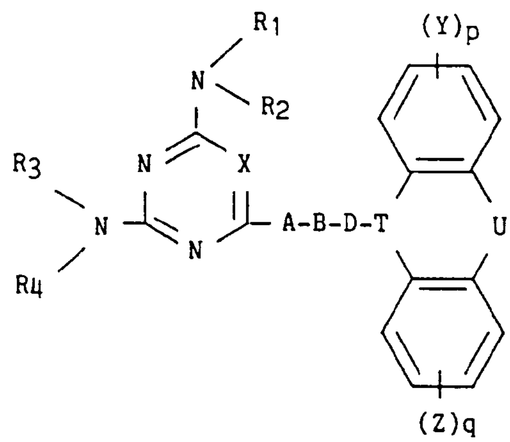

(I)

wherein :

- $R_1$, $R_2$, $R_3$ and $R_4$ are the same or different and each represents a hydrogen atom, a cycloalkyl radical having from 3 to 6 carbon atoms or a straight-chain or branched alkyl radical having from 1 to 6 carbon atoms, that optionally contains a double or a triple bond and is optionally substituted by a halogen atom, by one or more hydroxy radicals, or by an amino radical

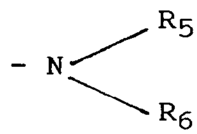

wherein $R_5$ and $R_6$ are the same or different and each represents a hydrogen atom or an alkyl radical having from 1 to 5 carbon atoms, or $R_5$ and $R_5$, together with the nitrogen atom to which they are bonded, form a pentagonal, hexagonal or heptagonal heterocycle optionally containing an oxygen or sulphur atom;
- X represents a nitrogen atom or the group CH;
- A represents a single bond, a straight-chain or branched hydrocarbon radical having from 1 to 3 carbon atoms, or a group -NH-A'- wherein A' represents a hydrocarbon chain having from 2 to 6 carbon atoms that optionally contains an oxygen or sulphur atom and is optionally substituted by a hydroxy radical;
- B represents a heterocyclic radical of formula :

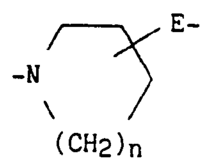

or

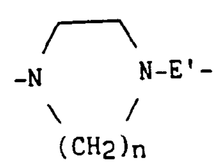

wherein :

. n represents an integer of from 1 to 3,
. E represents an oxygen or sulphur atom or an -NR- or $-CH_2NR-$ radical, R representing a hydrogen atom or an alkyl or alkenyl radical each having up to 5 carbon atoms, and
. E' represents a single bond or an -NR- radical as defined above;

- D represents a single bond or a straight-chain or branched hydrocarbon radical having up to 6 carbon atoms;
- T represents :

. a CR' radical wherein R' represents a hydrogen atom or an alkyl radical having from 1 to 5 carbon atoms,
. a

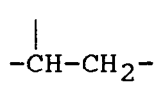

radical or
. a nitrogen atom;

- U represents :

. a single bond,
. a CHR" radical wherein R" represents a hydrogen atom or an alkyl radical having from 1 to 5 carbon atoms,
. a di- or tri-methylene radical [$(CH_2)_2$ and $(CH_2)_3$],
. a -CH=CH radical,
. an oxygen or sulphur atom,
. an -NR"' radical wherein R"' represents a hydrogen atom or an alkyl radical having from 1 to 5 carbon atoms,

. a radical of formula :
CO, $SO_2$, $-CH_2-O-$, $-CH_2-S-$, $-CH_2-SO_2-$,

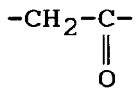,

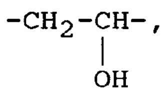, -CH=N-, 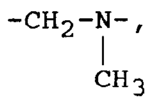, 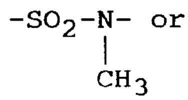 or 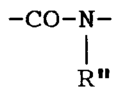,

wherein R''' is as defined hereinbefore; and

- furthermore, when T and U represent CR' and CHR'' respectively and R' and R'' are other than H, R' and R'' may together represent a polymethylene bridge having 2 or 3 carbon atoms;
- Y and Z are the same or different and each represents a hydrogen atom, a halogen atom, a trifluoromethyl radical, or an alkyl or alkoxy radical each having from 1 to 3 carbon atoms; and
- p and q are the same or different and are each 1 or 2;

and, when the general formula I contains one or more chiral carbon atoms, the corresponding enantiomers or diastereoisomers,
and their physiologically tolerble salts with appropriate acids,
characterised in that :

- either a compound of the general formula II :

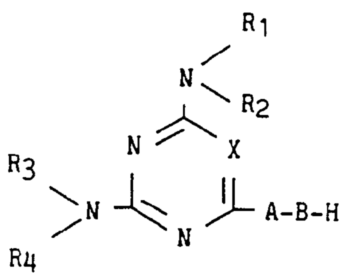

(II)

wherein $R_1$, $R_2$, $R_3$, $R_4$, X, A and B are as defined above, is condensed with a compound of the general formula III :

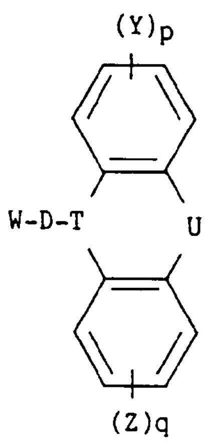

(III)

wherein D, T, U, Y, Z, p and q are as defined above and W represents a halogen atom or a tosyloxy radical;

- or a halogenated compound of the general formula IV :

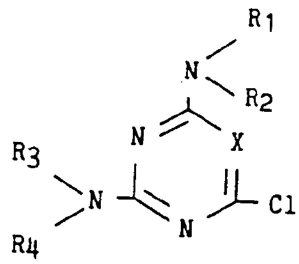

(IV)

wherein $R_1$, $R_2$, $R_3$, $R_4$ and X are as defined above, is condensed with a compound of the general formula V :

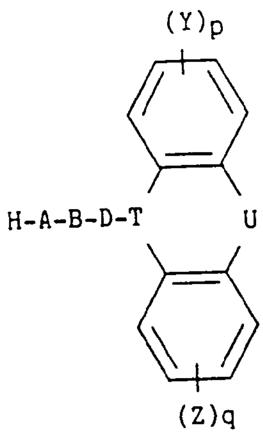

(V)

wherein A, B, D, T, U, Y, Z, p and q are as defined above,

and, if desired, the compounds of formula I so obtained are treated with physiologically tolerable acids to yield the corresponding acid addition salts.

**2.** A process for the preparation of the compounds of formula I defined in claim 1 and corresponding to the general formula I' :

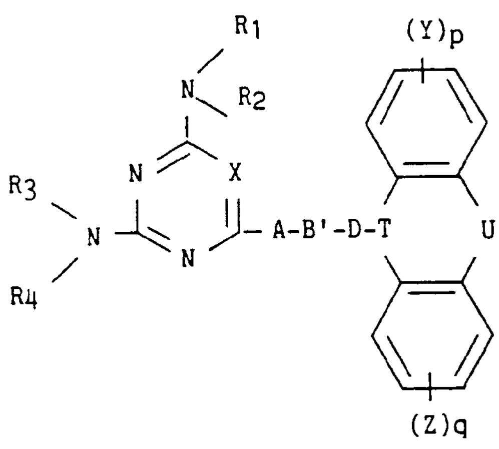

(I')

wherein :

- $R_1$, $R_2$, $R_3$, $R_4$, X, A, D, T, U, Y, Z, p and q are as defined in claim 1, and
- B' represents the radical :

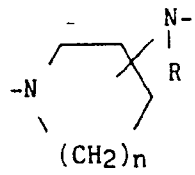

wherein n and R are as defined in claim 1, characterised in that

. a compound of the general formula VI :

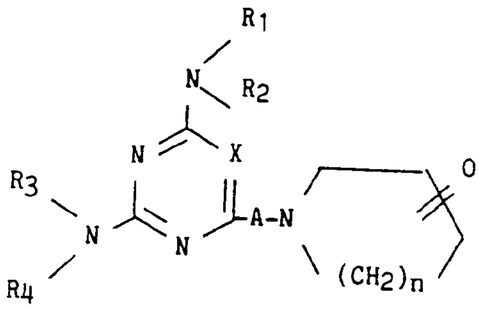

(VI)

wherein $R_1$, $R_2$, $R_3$, $R_4$, X, A and n are as defined in claim 1, is condensed

. with a compound of the general formula VII :

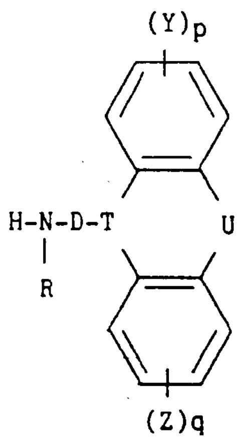

(VII)

wherein R, D, T, U, Y, Z, p and q are as defined above;

and, if desired, the compounds I' so obtained are treated with physiologically tolerable acids to yield the corresponding acid addition salts.